(19) Europäisches Patentamt / European Patent Office / Office européen des brevets

(11) **EP 3 394 014 B1**

(12) **FASCICULE DE BREVET EUROPEEN**

(45) Date de publication et mention de la délivrance du brevet:
**18.10.2023 Bulletin 2023/42**

(21) Numéro de dépôt: **16809095.9**

(22) Date de dépôt: **14.12.2016**

(51) Classification Internationale des Brevets (IPC):
*C07C 17/23* (2006.01)   *C07C 17/386* (2006.01)
*C07C 21/18* (2006.01)

(52) Classification Coopérative des Brevets (CPC):
(C-Sets disponibles)
**C07C 17/386; C07C 17/25;** C09K 5/045;
C09K 2205/126                                      (Cont.)

(86) Numéro de dépôt international:
**PCT/EP2016/080946**

(87) Numéro de publication internationale:
**WO 2017/108520 (29.06.2017 Gazette 2017/26)**

(54) **PROCEDE DE PRODUCTION ET DE PURIFICATION DU 2,3,3,3-TETRAFLUORO-1-PROPENE**

VERFAHREN ZUR HERSTELLUNG UND REINIGUNG VON 2,3,3,3-TETRAFLUOR-1-PROPEN

METHOD FOR PRODUCING AND PURIFYING 2,3,3,3-TETRAFLUORO-1-PROPENE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2015 FR 1563166**

(43) Date de publication de la demande:
**31.10.2018 Bulletin 2018/44**

(73) Titulaire: **Arkema France**
**92700 Colombes (FR)**

(72) Inventeurs:
• **BABA-AHMED, Abdelatif**
  **69190 Saint-fons (FR)**
• **COLLIER, Bertrand**
  **69230 Saint-genis-laval (FR)**
• **DEUR-BERT, Dominique**
  **69390 Charly (FR)**
• **WENDLINGER, Laurent**
  **69510 Soucieu En Jarrest (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**WO-A1-2009/105521     WO-A2-2008/030440**

(52) Classification Coopérative des Brevets (CPC):
(Cont.)

C-Sets
**C07C 17/25, C07C 21/18;**
**C07C 17/386, C07C 21/18**

**Description**

**Domaine technique de l'invention**

L'invention se rapporte à un procédé de purification du 2,3,3,3-tetrafluoro-1-propène. En outre, l'invention se rapporte également à un procédé de production et de purification du 2,3 ,3,3-tetrafluoro-1-propène.

**Arrière-plan technologique de l'invention**

Les hydrofluorocarbures (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. Les HFO ont été identifiés comme des alternatives souhaitables au HCFC du fait de leurs faibles valeurs d'ODP (Ozone Depletion Potential ou potentiel d'appauvrissement de la couche d'ozone) et de GWP (Global Warming Potential ou potentiel de réchauffement climatique).

La plupart des procédés de fabrication des hydrofluorooléfines font appel à une réaction de fluoration et/ou dehydrohalogénation. Ces réactions sont effectuées en phase gazeuse et génèrent des impuretés qu'il faut par conséquent éliminer pour obtenir le composé désiré dans un degré de pureté suffisant pour les applications visées.

Par exemple, dans le cadre de la production de 2,3,3,3-tétrafluoro-1-propène (HFO-1234yf), la présence d'impuretés telles que le 1-chloro-3,3,3-trifluoro-1-propene (1233zd), 1,3,3,3-tetrafluoro-1-propene (1234ze) et le 1,1,1,3,3-pentafluoropropane (245fa) sont observées. Ces impuretés sont des isomères des composés principaux visant à être obtenus par le procédé de production du 2,3,3,3-tétrafluoro-1-propène outre ce dernier, i.e. 2-chloro-3,3,3-trifluoro-1-propene (1233xf) et 1,1,1,2,2-pentafluoropropane (245cb). Compte tenu des points d'ébullition respectifs du 1-chloro-3,3,3-trifluoro-1-propene (1233zd), 1,3,3,3-tetrafluoro-1-propene (1234ze) et le 1,1,1,3,3-pentafluoropropane (245fa), ceux-ci peuvent s'accumuler dans le réacteur et ainsi empêcher la formation des produits d'intérêt.

La purification de ce type de mélange réactionnel peut être effectuée par différentes techniques connues de l'art antérieur, telles que par exemple la distillation. Cependant lorsque les composés à purifier ont des points d'ébullition trop proche ou lorsque ceux-ci forment des compositions azéotropes ou quasi-azéotropes, la distillation n'est pas un procédé efficace. Des procédés de distillation extractive ont ainsi été décrits.

On connaît par EP 0 864 554 un procédé de purification d'un mélange comprenant 1,1,1,3,3-pentafluoropropane (245fa) et 1-chloro-3,3,3-trifluoro-trans-1-propene (1233zd) par distillation en présence d'un solvant ayant un point d'ébullition supérieur à celui de 1-chloro-3,3,3-trifl uoro-trans-1-propene.

On connaît par WO 03/068716 un procédé de récupération de pentafluoroéthane à partir d'un mélange comprenant du pentafluoroéthane et du chloropentafluoroéthane par distillation en présence d'hexafluoropropène.

On connaît aussi par WO 98/19982 un procédé de purification du 1,1-difluoroéthane par distillation extractive. Le procédé consiste à mettre en contact un agent d'extraction avec un mélange de 1,1-difluoroéthane et de chlorure de vinyle. L'agent d'extraction est choisi parmi les hydrocarbures, les alcools, les chlorocarbures ayant un point d'ébullition compris entre 10°C et 120°C. Comme mentionné par WO 98/19982, la sélection de l'agent d'extraction peut s'avérer complexe en fonction des produits à séparer.

On connaît par WO 2009/105521 un procédé de purification par distillation azéotropique d'un mélange comprenant du 1,3,3,3-tetrafluoropropene et du 2,3,3,3-tetrafluoropropene. Il existe donc toujours un besoin pour la mise en oeuvre d'un procédé particulier de purification du 2,3,3,3-tetrafluoro-1-propène.

**Résumé de l'invention**

Dans un procédé de production du 2,3,3,3-tetrafluoro-1-propène, le choix de conditions opérationnelles particulières peut favoriser la présence de certaines impuretés ou d'isomères de celles-ci. La présence d'impuretés telles que 1,3,3,3-tetrafluoro-1-propene (1234ze) peut être observée tout comme celle de 1-chloro-3,3,3-trifluoro-1-propene (1233zd), et 1,1,1,3,3-pentafluoropropane (245fa). Ces impuretés peuvent provenir de réactions secondaires induites par des composés produits intermédiairement pendant la production du 2,3,3,3-tetrafluoro-1-propène, et peuvent présenter des propriétés physiques telles que leur élimination peut s'avérer complexe. La présente invention permet la production de 2,3,3,3-tetrafluoro-1-propène avec une pureté améliorée.

Selon un premier aspect, la présente invention fournit un procédé de purification du 2,3,3,3-tétrafluoro-1-propène (1234yf) à partir d'une première composition comprenant du 2,3,3,3-tétrafluoro-1-propène et au moins un des composés sélectionné parmi le groupe consistant en 1,1-difluoroéthane (152a), chloropentafluoroéthane (115), trans-1,3,3,3-tétrafluoro-1-propene (1234ze-E), ledit procédé comprenant les étapes de:

a) mise en contact de ladite première composition avec au moins un agent d'extraction organique pour former une seconde composition ;

b) distillation extractive de ladite seconde composition pour former :

    i) une troisième composition comprenant ledit agent d'extraction organique et ledit au moins un des composés sélectionné parmi le groupe consistant en 1,1-difluoroéthane (152a), chloropentafluoroéthane (115), trans-1,3,3,3-tetrafluoro-1-propene (1234ze-E); et

    ii) un courant comprenant le 2,3,3,3-tétrafluoro-1-propène,

c) récupération et séparation de ladite troisième composition pour former un courant comprenant ledit agent d'extraction organique et un courant comprenant ledit au moins un des composés sélectionné parmi le groupe consistant en 1,1-difluoroéthane (152a), chloropentafluoroéthane (115), trans-1,3,3,3-tetrafluoro-1-propene (1234ze-E) ; de préférence le courant comprenant ledit agent d'extraction organique peut être recyclé à l'étape a).

De préférence, le courant comprenant le 2,3,3,3-tétrafluoro-1-propène formé à l'étape b) est récupéré. Ce courant peut être éventuellement purifié pour atteindre un degré de pureté adapté à des spécifications commerciales particulières.

De préférence, ledit agent d'extraction organique peut avoir un point d'ébullition compris entre 10 et 150°C.

Selon un mode de réalisation préféré, la première composition comprend 2,3,3,3-tétrafluoro-1-propène, trans-1,3,3,3-tetrafluoro-1-propene (1234ze-E) et optionnellement ou non au moins un des composés sélectionné parmi le groupe consistant en 1,1-difluoroéthane (152a) et chloropentafluoroéthane (115) et la troisième composition comprend ledit agent d'extraction organique, trans-1,3,3,3-tetrafluoro-1-propene (1234ze-E) et optionnellement ou non au moins un des composés sélectionné parmi le groupe consistant en 1,1-difluoroéthane (152a) et chloropentafluoroéthane (115) ; l'étape c) étant la récupération de ladite troisième composition et séparation entre d'autre part ledit agent d'extraction organique et d'autre part le trans-1,3,3,3-tetrafluoro-1-propene (1234ze-E) et optionnellement ou non au moins un des composés sélectionné parmi le groupe consistant en 1,1-difluoroéthane (152a) et chloropentafluoroéthane (115).

Selon un mode de réalisation préféré, ledit agent d'extraction organique est un solvant choisi parmi le groupe consistant en hydrocarbure, hydrohalocarbure, alcool, cétone, amine, ester, éther, aldéhyde, nitrile, carbonate, thioalkyle, amide et hétérocycle ; ou ledit agent d'extraction organique est difluorodiethylsilane, triethylfluorosilane ou l'acide perfluorobutanoïque; de préférence parmi le groupe consistant en amine, éther, cétone, ester, alcool, aldéhyde, hétérocycle.

Selon un mode de réalisation préféré, ledit agent d'extraction organique a un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle

    $\gamma_{1,S}$ représente le coefficient d'activité du 2,3,3,3-tétrafluoro-1-propène dans ledit agent d'extraction organique à dilution infinie,

    P1 représente la pression de vapeur saturante du 2,3,3,3-tétrafluoro-1-propène,

    $y_{2,s}$ représente le coefficient d'activité dudit au moins un des composés consistant en 1,1-difluoroéthane (152a), chloropentafluoroéthane (115), trans-1,3,3,3-tetrafluoro-1-propene (1234ze-E) dans ledit agent d'extraction organique à dilution infinie, avantageusement le coefficient d'activité du trans-1,3,3,3-tetrafluoro-1-propene (1234ze-E) dans ledit agent d'extraction organique à dilution infinie,

    P2 représente la pression de vapeur saturante de dudit au moins un des composés consistant en 1,1-difluoroéthane (152a), chloropentafluoroéthane (115), trans-1,3,3,3-tetrafluoro-1-propene (1234ze-E), avantageusement le coefficient d'activité du trans-1,3,3,3-tetrafluoro-1-propene (1234ze-E) ;

    avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

Selon un mode de réalisation préféré, ledit agent d'extraction organique a une capacité d'absorption $C_{2,S}$ supérieure ou égale à 0,20, ladite capacité d'absorption étant calculé par la formule $C_{2,S} = 1/(\gamma_{2,S})$ dans laquelle $\gamma_{2,S}$ représente le coefficient d'activité dudit au moins un des composés consistant en 1,1-difluoroéthane (152a), chloropentafluoroéthane (115), trans-1,3,3,3-tetrafluoro-1-propene (1234ze-E) dans ledit agent d'extraction organique à dilution infinie, avantageusement le coefficient d'activité du trans-1,3,3,3-tetrafluoro-1-propene (1234ze-E) ; avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 1,0.

Selon un mode de réalisation préféré, ladite première composition est une composition azéotropique ou quasi-azéotropique comprenant 2,3,3,3-tetrafluoro-1-propène et ledit au moins un des composés sélectionné parmi le groupe consistant en 1,1-difluoroéthane (152a), chloropentafluoroéthane (115), trans-1,3,3,3-tetrafluoro-1-propene (1234ze-E), de préférence une composition azéotropique ou quasi-azéotropique comprenant 2,3,3,3-tetrafluoro-1-propène, trans-1,3,3,3-tetrafluoro-1-propene (1234ze-E) et optionnellement ou non au moins un des composés sélectionné parmi le groupe consistant en 1,1-difluoroéthane (152a), chloropentafluoroéthane (115).

Selon un mode de réalisation préféré, ledit agent d'extraction organique est choisi parmi le groupe consistant en éthylamine, isopropylamine, diethylether, ethoxy-ethene, dimethoxymethane, n-propylamine, methyl-t-butylether, diethylamine, propanone, methylacetate, isobutanal, tetrahydrofurane, isopropylformate, diisopropylether, 2-ethoxy-2-methyl-propane, ethylacetate, butanone, diethoxymethane, isopropylacetate, 3-pentylamine, 2-methoxyethanamine, tert-butylacetate, dioxane, 3-pentanone, 1,1-diethoxyethane, 2-pentanone, trimethoxymethane, n-pentylamine, 1,3-dioxane, 3,3-dimethyl-2-butanone, sec-butylacetate, 4-methyl-2-pentanone, 1,2-diaminoethane, 1-methoxy2-propanol, diethylcarbonate, n-butylacetate, 1-ethoxy-2-propanol, hexanal ; avantageusement ledit agent d'extraction organique est choisi parmi le groupe consistant en éthylamine, isopropylamine, diethylether, dimethoxymethane, n-propylamine, , diethylamine, , diisopropylether, 2-ethoxy-2-methyl-propane, butanone, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal ; de préférence ledit agent d'extraction organique est choisi parmi le groupe consistant en éthylamine, isopropylamine, diethylether, dimethoxymethane, n-propylamine,, diethylamine,, diisopropylether, 2-ethoxy-2-methyl-propane, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal..

De préférence, le courant comprenant le 2,3,3,3-tetrafluoro-1-propène formé à l'étape b) est récupéré et est dépourvu d'au moins un des composés sélectionné parmi le groupe consistant en 1,1-difluoroéthane (152a), chloropentafluoroéthane (115), trans-1,3,3,3-tetrafluoro-1-propene (1234ze-E), de préférence est dépourvu de trans-1,3,3,3-tetrafluoro-1-propene (1234ze-E).

Selon un mode de réalisation préféré, le procédé comprend préalablement à l'étape a) les étapes :

i') mise en oeuvre d'une composition comprenant 2,3,3,3-tetrafluoro-1-propène, des impuretés ayant un point d'ébullition inférieur au point d'ébullition du 2,3,3,3-tetrafluoro-1-propène, et au moins un des composés sélectionné parmi le groupe consistant en 1,1-difluoroéthane (152a), chloropentafluoroéthane (115), trans-1,3,3,3-tetrafluoro-1-propene (1234ze-E), et optionnellement des impuretés lourdes ;

ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E), et optionnellement des impuretés lourdes, récupéré en bas de colonne de distillation ;

iii') optionnellement, distillation dudit premier courant récupéré en bas de colonne de distillation à l'étape ii') pour récupérer en tête de colonne un second courant comprenant du 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E), et en bas de colonne de distillation un courant comprenant les impuretés lourdes ;

ledit premier courant récupéré à l'étape ii') ou ledit second courant récupéré à l'étape iii') correspond à ladite première composition mise en oeuvre à l'étape a).

Les impuretés lourdes peuvent contenir par exemple 1,1,1,3,3,3-hexafluoropropane (236fa), 1,1,1,2,3,3-hexafluoropropane (236ea), 1,1,1,2,3,3,3-heptafluoropropane (227ca), cis-1,3,3,3-tetrafluoro-1-propene (1234ze-Z), des dimères ou des trimères issus de l'un des composés présents dans la composition ou le courant considéré, par exemple des hydrocarbures en C4 ou C5 tels que hexafluorobutène (1336), heptafluorobutène (1327), octafluorobutane (338), nonafluoropentène (1429), heptafluoropentène (1447).

Selon un second aspect de la présente invention, un procédé de production de 2,3,3,3-tetrafluoro-1-propène est fourni. Ledit procédé comprend les étapes de :

A) fluoration en présence d'un catalyseur d'un composé de formule $CX(Y)_2-CX(Y)_m-CH_mXY$ (I) dans laquelle X et Y représentent indépendamment H, F, ou Cl et m = 0 ou 1 ; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E) ;

C) mise en oeuvre du procédé de purification du 2,3,3,3-tetrafluoro-1-propène selon la présente invention à partir du courant récupéré à l'étape B).

Selon un autre aspect, la présente demande décrit une composition comprenant du 2,3,3,3-tetrafluoropropène, trans-1,3,3,3-tetrafluoro-1-propene (1234ze-E) et un agent d'extraction organique sélectionné parmi le groupe consistant en éthylamine, isopropylamine, diethylether, dimethoxymethane, n-propylamine, , diethylamine, , diisopropylether, 2-ethoxy-2-methyl-propane, butanone, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal.

Selon un mode de réalisation préféré, pour l'une quelconque des compositions ci-dessus, l'agent d'extraction organique peut être sélectionné parmi le groupe consistant en éthylamine, isopropylamine, diethylether, dimethoxymethane, n-propylamine, , diethylamine, diisopropylether, 2-ethoxy-2-methyl-propane, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal.

**Brève description des figures**

Les figures 1a-c représentent schématiquement un dispositif mettant en oeuvre un procédé de purification du 2,3,3,3-tetrafluoro-1-propène selon un mode de réalisation particulier de la présente invention.

La figure 2 représente schématiquement un dispositif mettant en oeuvre un procédé de production du 2,3,3,3-tetrafluoro-1-propène selon un mode de réalisation particulier de la présente invention.

**Description détaillée de l'invention**

Le terme « hydrocarbure » tel qu'utilisé ici se réfère à des composés linéaires ou branchés d'alcane en $C_1$-$C_{20}$, cycloalcane en $C_3$-$C_{20}$, alcène en $C_2$-$C_{20}$, cycloalcène en $C_3$-$C_{20}$, arène en $C_6$-$C_{18}$. Par exemple, le terme alcane se réfère à des composés de formule $C_nH_{2n+2}$ dans lequel n est compris entre 1 et 20. Le terme alcane en $C_1$-$C_{20}$ englobe par exemple le pentane, hexane, heptane, octane, nonane, décane ou des isomères de ceux-ci. Le terme alcène en $C_2$-$C_{20}$ se réfère à des composés hydrocarbonés comprenant une ou plusieurs doubles liaisons carbone-carbone et comprenant de 2 à 20 atomes de carbone. Le terme cycloalcane en $C_3$-$C_{20}$ se réfère à un cycle hydrocarboné saturé comprenant de 3 à 20 atomes de carbone. Le terme aryle en $C_6$-$C_{18}$ se réfère à des composés hydrocarbonés cycliques et aromatiques comprenant de 6 à 18 atomes de carbone. Le terme cycloalcène en $C_3$-$C_{20}$ se réfère à des composés hydrocarbonés cycliques comprenant de 3 à 20 atomes de carbone et comprenant une ou plusieurs doubles liaisons carbone-carbone.

Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « alkényle » désigne un radical monovalent de 2 à 20 atomes de carbone et au moins une double liaison carbone-carbone. Le terme « alkynyle » désigne un radical monovalent de 2 à 20 atomes de carbone et au moins une triple liaison carbone-carbone. Le terme « halogène » se réfère à un groupement -F, -Cl, -Br ou -I. Le terme « cycloalkényle » se réfère à un radical monovalent issu d'un cycloalcène comprenant de 3 à 20 atomes de carbone. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -NR$^a$C(O)R$^b$, -C(O)NR$^a$R$^b$, -CN, -NO$_2$, -NR$^a$R$^b$, -OR$^a$, -SR$^a$, -CO$_2$R$^a$, -OC(O)OR$^a$, -OC(O)R$^a$, - C(O)H, -C(O)R$^a$, dans lequel R$^a$ et R$^b$ sont indépendamment l'un de l'autre hydrogène, alkyle en $C_1$-$C_{20}$ non substitué, alkényle en $C_2$-$C_{20}$ non substitué, alkynyle en $C_2$-$C_{20}$ non substitué, cycloalkyle en $C_3$-$C_{20}$ non substitué, cycloalkényle en $C_3$-$C_{20}$ non substitué, aryle en $C_6$-$C_{18}$ non substitué. Dans les substituants -NR$^a$R$^b$, -NR$^a$C(O)R$^b$, -C(O)NR$^a$R$^b$, R$^a$ et R$^b$ peuvent former avec l'atome d'azote ou avec le groupement fonctionnel auquel ils sont rattachés un hétérocycle saturé ou insaturé, aromatique ou non, comprenant de 4 à 10 chainons.

Le terme « hydrohalocarbures » se réfère à des composés de formule R$^a$X dans laquelle R$^a$ est sélectionné parmi alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ et X représente un atome de chlore, de fluor, de brome ou d'iode. Les substituants alkyle en $C_1$-$C_{20}$, alkényle en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ peuvent être substitués ou non par un ou plusieurs substituants -OH, halogène, -NR$^a$C(O)R$^b$, -C(O)NR$^a$R$^b$ -CN, -NO$_2$, -NR$^a$R$^b$, -OR$^a$, - SR$^a$, -CO$_2$R$^a$, -OC(O)OR$^a$, -OC(O)R$^a$, -C(O)H, -C(O)R$^a$, dans lequel R$^a$ et R$^b$ sont tels que définis ci-dessus.

Le terme « alcool » se réfère à des hydrocarbures ou hydrohalocarbures tels que définis ci-dessus dans lequel au moins un atome d'hydrogène est remplacé par un groupement hydroxyle -OH.

Le terme « cétone » se réfère à des hydrocarbures comprenant au moins un ou plusieurs groupements fonctionnels carbonyle R$^C$-C(O)-R$^d$ dans lequel R$^c$ et R$^d$ sont indépendamment l'un de l'autre un alkyle en $C_1$-$C_{20}$, alkényle

en $C_2$-$C_{20}$, alkynyle en $C_2$-$C_{20}$, cycloalkyle en $C_3$-$C_{20}$, cycloalkényle en $C_3$-$C_{20}$, aryle en $C_6$-$C_{18}$ et peuvent être substitués ou non par un ou plusieurs substituants -OH, halogène, -NR$^a$C(O)R$^b$, -C(O)NR$^a$R$^b$ -CN, -NO$_2$, -NR$^a$R$^b$, -OR$^a$, -SR$^a$, -CO$_2$R$^a$, - OC(O)OR$^a$, -OC(O)R$^a$, -C(O)H, -C(O)R$^a$, dans lequel R$^a$ et R$^b$ sont tels que définis ci-dessus, R$^c$ et R$^d$ pouvant être liés entre eux pour former avec le groupement carbonyle auquel ils sont rattachés une cétone cyclique comprenant de 4 à 10 chainons, de préférence de 4 à 7 chainons. La cétone cyclique peut également comprendre une ou plusieurs doubles liaisons carbone-carbone. La cétone cyclique peut également être substituée ou non par un ou plusieurs substituants tels que définis ci-dessus.

Le terme « amine » se réfère à des hydrocarbures comprenant au moins un ou plusieurs groupements fonctionnels amine -NR$^c$R$^c$ dans lequel R$^c$ et R$^d$ sont tels que définis ci-dessus, R$^c$ et R$^d$ pouvant être liés entre eux pour former avec l'atome d'azote auquel ils sont rattachés un hétérocycle aromatique ou non comprenant de 4 à 10 chainons.

Le terme « esters » se réfère à des composés de formule R$^c$-C(O)-O-R$^d$ dans lequel R$^c$ et R$^d$ sont tels que définis ci-dessus, R$^c$ et R$^d$ pouvant être liés entre eux pour former avec le groupement ester un cycle comprenant de 4 à 20 atomes de carbone.

Le terme « éther » se réfère à des composés de formule R$^c$-O-R$^d$ dans lequel R$^c$ et R$^d$ sont tels que définis ci-dessus, R$^c$ et R$^d$ pouvant être liés entre eux pour former avec l'atome d'oxygène auquel ils sont rattachés un hétérocycle comprenant de 4 à 20 atomes de carbone.

Le terme « aldéhyde » se réfère à des composés comprenant au moins un ou plusieurs groupements fonctionnels -C(O)-H.

Le terme « nitrile » se réfère à des composés comprenant au moins un ou plusieurs groupements fonctionnels -CN.

Le terme « carbonate » se réfère à des composés de formule R$^c$-O-C(O)-O-R$^d$ dans lequel R$^c$ et R$^d$ sont tels que définis ci-dessus.

Le terme « thioalkyle » concerne des composés de formule R$^c$SR$^d$ dans laquelle R$^c$ et R$^d$ sont tels que définis ci-dessus.

Le terme « amide » concerne des composés de formule R$^c$C(O)NR$^e$R$^d$ dans laquelle R$^c$ et R$^d$ sont tels que définis ci-dessus, R$^e$ ayant la même définition que R$^c$, R$^c$ et R$^d$ pouvant être liés entre eux pour former avec le groupement amide -C(O)N- auquel ils sont rattachés une amide cyclique comprenant de 4 à 10 chainons, de préférence de 4 à 7 chainons. L'amide cyclique peut également comprendre une ou plusieurs doubles liaisons carbone-carbone. L'amide cyclique peut également être substituée ou non par un ou plusieurs substituants tels que définis ci-dessus.

Le terme « hétérocycle » désigne un cycle carboné comprenant de 4 à 10 chaînons dont au moins un des chaînons est un hétéroatome sélectionné parmi le groupe consistant en O, S, P et N. L'hétérocycle peut comprendre un ou plusieurs double liaison carbone-carbone ou une ou plusieurs double liaison carbone-hétéroatome ou une ou plusieurs double liaison hétéroatomehétéroatome. De préférence, l'hétérocycle peut comprendre 1, 2, 3, 4 ou 5 hétéroatomes tel que défini ci-dessus. En particulier, l'hétérocycle peut comprendre 1, 2 ou 3 hétéroatomes sélectionné parmi l'oxygène, l'azote ou le soufre. De préférence, l'hétérocycle peut être un cycle carboné comprenant de 4 à 6 chaînons dont 1, 2 ou 3 chaînons sont des hétéroatomes sélectionnés parmi O ou N. L'hétérocycle peut être ou non substitué par un ou plusieurs substituants choisi parmi -OH, halogène, -NR$^a$C(O)R$^b$, -C(O)NR$^a$R$^b$ -CN, -NO$_2$, -NR$^a$R$^b$, -OR$^a$, -SR$^a$, - CO$_2$R$^a$, -OC(O)OR$^a$, -OC(O)R$^a$, -C(O)H, -C(O)R$^a$ dans lequel R$^a$ et R$^b$ sont tels que définis ci-dessus.

Le terme « composition azéotropique » désigne un mélange liquide de deux ou plusieurs composés se comportant comme une substance unique, et qui bout à température fixe en gardant une composition en phase liquide identique à celle de la phase gaz. Le terme « composition quasi-azéotropique » désigne un mélange liquide de deux ou plusieurs composés ayant un point d'ébullition constant ou qui a tendance à ne pas se fractionner lorsqu'il est soumis à l'ébullition ou l'évaporation.

Le terme « agent d'extraction organique » se réfère à un composé comprenant au moins un atome de carbone.

Selon un premier aspect, l'invention se rapporte à un procédé de purification du 2,3,3,3-tetrafluoro-1-propene (1234yf). Le procédé de purification est effectué à partir d'une première composition comprenant du 2,3,3,3-tetrafluoro-1-propène et au moins un des composés sélectionné parmi le groupe consistant en 1,1-difluoroéthane (152a), chloropentafluoroéthane (115), trans-1,3,3,3-tetrafluoro-1-propene (1234ze-E).

De préférence, ledit procédé comprend les étapes de :

   a) mise en contact de ladite première composition avec au moins un agent d'extraction organique pour former une seconde composition ;
   b) distillation extractive de ladite seconde composition pour former :

      i) une troisième composition comprenant ledit agent d'extraction organique et ledit au moins un des composés sélectionné parmi le groupe consistant en 1,1-difluoroéthane (152a), chloropentafluoroéthane (115), trans-1,3,3,3-tetrafluoro-1-propene (1234ze-E), de préférence une troisième composition comprenant ledit agent d'extraction organique et trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115); et

ii) un courant comprenant le 2,3,3,3-tétrafluoro-1-propène,

c) récupération et séparation de ladite troisième composition pour former un courant comprenant ledit agent d'extraction organique et un courant comprenant ledit au moins un des composés sélectionné parmi le groupe consistant en 1,1-difluoroéthane (152a), chloropentafluoroéthane (115), trans-1,3,3,3-tetrafluoro-1-propene (1234ze-E) ; de préférence récupération et séparation de ladite troisième composition pour former un courant comprenant ledit agent d'extraction organique et un courant comprenant trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115).

De préférence, ledit agent d'extraction organique est recyclé à l'étape a).

De préférence, ledit courant ii) comprenant le 2,3,3,3-tétrafluoropropène obtenu à l'étape b) est récupéré.

Ladite première composition peut comprendre du 2,3,3,3-tétrafluoro-1-propène et au moins deux ou l'ensemble des composés sélectionné parmi le groupe consistant en 1,1-difluoroéthane (152a), chloropentafluoroéthane (115), trans-1,3,3,3-tetrafluoro-1-propene (1234ze-E).

De préférence, la première composition comprend 2,3,3,3-tétrafluoro-1-propène, trans-1,3,3,3-tetrafluoro-1-propene (1234ze-E) et optionnellement ou non au moins un des composés sélectionné parmi le groupe consistant en 1,1-difluoroéthane (152a), chloropentafluoroéthane (115).

Ladite première composition peut comprendre entre 75 et 99,99 % en poids de 2,3,3,3-tétrafluoro-1-propène sur base du poids total de la première composition, avantageusement entre 80 et 99,9% en poids, de préférence entre 85 et 99,8% et en particulier entre 90 et 99,5% en poids de 2,3,3,3-tétrafluoro-1-propène sur base du poids total de la première composition.

Lorsqu'elle en contient, ladite première composition peut comprendre moins de 2000 ppm poids de 1,1-difluoroéthane (152a) sur base du poids total de la première composition, avantageusement entre 1 et 1000 ppm poids, de préférence entre 1 et 500 ppm et en particulier entre 1 et 250 ppm en poids de 1,1-difluoroéthane (152a) sur base du poids total de la première composition.

Lorsqu'elle en contient, ladite première composition peut comprendre moins de 25 % en poids de trans-1,3,3,3-tetrafluoro-1-propene (1234ze-E) sur base du poids total de la première composition, avantageusement entre 0.1 et 20% en poids, de préférence entre 0.2 et 15% et en particulier entre 0.5 et 10% en poids, et plus particulièrement entre 3 et 10% en poids de trans-1,3,3,3-tetrafluoro-1-propene (1234ze-E) sur base du poids total de la première composition.

Lorsqu'elle en contient, ladite première composition peut comprendre moins de 2000 ppm poids de chloropentafluoroéthane (115) sur base du poids total de la première composition, avantageusement entre 1 et 1000 ppm poids, de préférence entre 1 et 500 ppm et en particulier entre 1 et 250 ppm en poids de chloropentafluoroéthane (115) sur base du poids total de la première composition.

Selon un mode de réalisation particulier, ledit agent d'extraction organique est un solvant choisi parmi le groupe consistant en hydrocarbure, hydrohalocarbure, alcool, cétone, amine, ester, éther, aldéhyde, nitrile, carbonate, thioalkyle, amide et hétérocycle. Alternativement, ledit agent d'extraction organique peut être difluorodiethylsilane, triethylfluorosilane ou l'acide perfluorobutanoïque. Avantageusement, ledit agent d'extraction organique est un solvant choisi parmi le groupe consistant en amine, éther, cétone, ester, alcool, aldéhyde, hétérocycle. De préférence, l'hétérocycle peut être un cycle carboné comprenant de 4 à 6 chaînons dont 1, 2 ou 3 chaînons sont des hétéroatomes sélectionnés parmi O ou N.

De préférence, les hydrocarbures sont sélectionnés parmi le groupe consistant en 2,2-dimethylbutane, 3-methylpentane, hexane, 2,4-dimethylpentane, cyclohexane, cyclohexene, methylcyclohexane, 2,4,4-trimethyl-1-pentene, 2,2,4-trimethyl-2-pentene, 1-methylcyclohexene, toluene, 2,3-dimethylhexane, octane, ethylcyclohexane, ethylbenzene, 1,4-dimethylbenzene, 1,2,3-trimethylcyclohexane, 1,2-dimethylbenzene, styrène.

De préférence, les hydrohalocarbures sont sélectionnés parmi le groupe consistant en Chloroethane 3-chloro-1,1,1,2,2,3-hexafluoropropane, 1,1,1,2,2,3,3,4,4-nonafluorobutane, 2-chloro-1,1,1,3,3,3-hexafluoropropane, bromofluoromethane, 1-bromo-1,2-difluoroethylene, 1-chloro-1,1,2,3,3,3-hexafluoropropane, 1-chloro-1,1,2,2-tetrafluoropropane, 1-chloro-1,1,2,2,3,3-hexafluoropropane, 2-chloro-1,1,1,2-tetrafluoropropane, 1,1,1,2,3-pentafluoropropane, trichlorofluoromethane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 1,1-dichloroethylene , 1,1,1,2,3,4,4,4-octafluorobutane, 2-chloro-2-fluoropropane, 1-chloro-2,2-difluoroethane, 2-chloropropane, bromoethane, dichloromethane, 2-chloro-1,1,1,3,3-pentafluoropropane, 1-chloro-1,2,2-trifluoropropane, iodomethane, 2,2-dichloro-1,1,1,3,3-pentafluoropropane, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, 1,1,2-trichloro-1,2,2-trifluoroethane, 1,2-dichloro-1,1,3,3,3-pentafluoropropane, 1-chloro-1,2,2,3-tetrafluoropropane, perfluorocyclohexane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 1,3-dichloro-1,1,2,3,3-pentafluoropropane, 1,2-dichloro-3,3,3-trifluoropropene, 1,3-dichloro-1,1,2,2,3-pentafluoropropane, 1,1,1,2,2,3,4,5,5,5-decafluoropentane, 2,2-dichloro-1,1,1,3-tetrafluoropropane, 1-chloro-2,2-difluoropropane, perfluoro-n-hexane, 1,1-dichloroethane, 2-bromopropane, 1,2-dichloro-1,2,3,3,4,4-hexafluorocyclobutane, 1,1-dichloro-2,2-difluoroethane, 2,2-dichloro-1,1,3,3-tetrafluoropropane, trichloromethane, 2,3-dichloro-1,1,1,2-tetrafluoropropane, 1,1-dich loro-1,2,2,3-tetrafluoropropane, 1,3-dichloro-1,1,2,2-tetrafluoropropane, 1,1,1-trichloro-2,2-difluoroethane, 1,2-dichloro-1,2,3,3-tetrafluoropropane, chlorobromomethane, 2-chlorobutane, 1,3-dichloro-1,1,3,3-tetrafluoropropane, 1,3-dichloro-1,2,2,3-tetrafluoropropane, 1,2-dichloro-1,1,2,3-tetrafluoropropane, 3-bromopropene, 2,3-dichloro-1,1,1,3-tetrafluoropropane, 1,1-dichloro-2-fluoroethane, 1-bromopropane, 1,1-difluoro-1,2,2-trichloroethane, 1,1,2-trichloro-1,2-difluoroethane, iodoethane, 2-bromo-2-methylpropane, 1,2-dichloro-1-fluoroethane, 1,1,1-trichloroethane, 1-chloro-3-fluoropropane, 2,3-dichloro-1,1,1-trifluoropropane, perfluoromethylcyclohexane, tetrachloromethane, 1-chlorobutane, perfluoroheptane, 1,3-dichloro-1,1,2-trifluoropropane, 1,1-dichloro-2,2,3-trifluoropropane, 1,3,3-trichloro-1,1,2,2-tetrafluoropropane, 2-chloro-2-methylbutane, 1,1,1-trichloro-2,2,3,3-tetrafluoropropane, 1,1,3-trichloro-1,2,2,3-tetrafluoropropane, 1,3-dichloro-1,2,2-trifluoropropane, trichloroethene, 1,1-dichloropropane, 1,2-dichloro-2-fluoropropane, 2-iodopropane, dichlorobromomethane, 2-bromobutane, 2,2-difluorotetrachloroethane, 1,1,2,2-tetrachloro-1,2-difluoro-ethane, 1-fluorohexane, 1,3-dichloro-1,2,3-trifluoropropane, 2,3-dichloro-1-propene, 1,2-dichloropropane, 3-chloropentane, trichloroacetaldehyde, isoamylchloride, 1,1,1-trichloro-2,2,3-trifluoropropane, 1-chloro-4-fluorobutane, 1-bromo-3-fluoropropane, 1-bromobutane, 1,1,2-trichloro-2-fluoroethane, 1-iodopropane, 1,1,3-trichloro-1,2,2-trifluoropropane, 1,1,3-trichloro-2,2,3-trifluoropropane, cis-1,3-dichloropropene, 2,2-dichlorobutane, bromotrichloromethaneperfluorooctane, 1,1,1,2-tetrachloro-2-fluoroethane, 1-bromo-2-chloroethane, 2-bromo-2-methylbutane, trans-1,3-dichloropropene, 2-fluorotoluene, 1,1,1-trichloro-3-fluoropropane, 3,3,3-trichloro-1-propene, 1-chloro-3,3-dimethylbutane, 1,1,1,2-tetrachloro-3,3,3-trifluoropropane, 2-bromopentane, trichloroacetylchloride, 2,3-dichlorobutane, 1,1,3,3-tetrachloro-1,2,2-trifluoropropane, 1,1,1,3-tetrachloro-2,2,3-trifluoropropane, 1-bromo-3-methylbutane, 1,3-dichloro-trans-2-butene, 1,3-dichloropropane, tetrachloroethene, 1,2-dibromo-1-fluoroethane, 1,2,2-trichloropropane, 1,2-dichlorobutane, perfluorononane, 1,2,2,3-tetrachloro-3,3-difluoropropane, 2,3-dichloro-2-methylbutane, 1-bromopentane, 1,2-dichloro-2-butene, 1-iodobutane, 1,2-dibromoethane, chlorobenzene, perfluorooctylbromide, 1,1,2-trichloropropane, 1,3-dichlorobutane, pentachlorofluoroethane, 1,2-dibromopropane, 1,2,3-trichloropropene, 1-chloro-3-bromopropane, perfluoro-n-decane, 1,1,3,3-tetrachloro-1-fluoropropane, 1,1,2,2,3-pentchloro-3,3-difluoropropane, 1,1,2,2-tetrachloroethane, 1,2-dichloropentane, tribromomethane.

De préférence, les alcools sont sélectionnés parmi le groupe consistant en méthanol, 2,2,2-trifluoroethanol, 1,1,1-trifluoro-2-propanol, ethanol, pentafluoro-1-propanol, 2-propanol, tert-butanol, 2,2-difluoroethanol, propanol, 2-allyloxyethanol, 2-butanol, 2-methyl-2-butanol, isobutanol, 2,2,3,3-tetraflouro-1-propanol, 2,2-dimethyl-1-propanol, 3-pentanol, 1-butanol, 1-methoxy2-propanol, 1-(dimethylamino)-2-propanol, 3-methyl-3-pentanol, 1-chloro-2-methyl-2-propanol, 4,4,4-trifluorobutanol, 3-fluoropropanol, 2-chloroethanol, 2-methoxyl-propanol, 1-ethoxy-2-propanol, 4-methyl-2-pentanol, 1,2-octanediol, 2-chloro-1-propanol, 2-(dimethylamino)-ethanol, 3-hexanol, 2-hexanol, 2-ethoxy-1-propanol, 1-pentanol, 3,3,4,4,5,5,6,6-octafluoro-1-pentanol, 2,3-dimethylbutanol, 2-ethyl-1-butanol, 2-methyl-1-pentanol, 2-propoxyethanol, 1-propoxy-2-propanol, 2-aminophenol.

De préférence, les cétones sont sélectionnées parmi le groupe consistant en 1,1,1-trifluoro-2-propanone, propanone, butanone, 3-pentanone, 2-pentanone, 3,3-dimethyl-2-butanone, 4-methyl-2-pentanone, 2-hexanone, 5-hexen-2-one, 4-methyl-2-hexanone.

De préférence, les amines sont sélectionnées parmi le groupe consistant en Ethylamine, isopropylamine, ethylmethylamine, 2-amino-2-methylpropane, n-propylamine, isopropylmethylamine, diethylamine, 2-butanamine, n-methylpropylamine, 1-butylamine, diisopropylamine, 3-methyl-2-butanamine, 3-pentylamine, n-methylbutylamine, 1-methoxy-2-propanamine, 2-methoxyethanamine, 2-methoxy-lpropanamine, n-pentylamine, n-methylhydroxylamine, dipropylamine, 2-ethoxyethanamine, n-methyl-1,2-ethanediamine, pyridine, 1,2-diaminoethane, 1,2-propanediamine, 2-ethylbutylamine, n-ethylethylenediamine, 1,1-diethoxy-n,n-dimethylmethanamine, 2-methylpyridine, 4-methyl-2-hexanamine, hexylamine, cyclohexylamine, n-ethyl-2-dimethylaminoethylamine, 1,3-propanediamine, 2-heptanamine, n,n-diethylethylenediamine, 2,6-dimethylpyridine, 4-methylpyridine, n,n'-diethyl-1,2-ethanediamine, dimethylethanolamine.

De préférence, les esters sont sélectionnés parmi le groupe consistant en Methylformate, methylacetate, isopropylformate, ethylacetate, n-propylformate, isopropylacetate, tert-butylacetate, ethylpropionate, sec-butylacetate, diethylcarbonate, n-butylacetate, bromoaceticacidmethylester, methylhexanoate.

De préférence, les éthers sont sélectionnés parmi le groupe consistant en 2,2,2-trifluoroethylmethylether, 1,1,2,2-tetrafluoroethylmethylether, 2-methoxy-1-propene, heptafluoro-1-methoxypropane, diethylether, ethoxy-ethene, dimethoxymethane, methylcyclopropylether, 2-ethoxy-propane, methyl-t-butylether, ethyl1,1,2,2-tetrafluoroethylether, chloromethoxymethane, diisopropylether, 2-ethoxy-2-methyl-propane, 2-ethoxy-butane, 1-methoxy-2-methyl-butane, 2,2-dimethoxypropane, 1-ethoxy-2-methylpropane, 1,2-dimethoxyethane, diethoxymethane, di-n-propylether, 1-ethoxy-butane, 1-methoxy-pentane, 1,2-dimethoxypropane, isopropyl-isobutyl-ether, 1,1-diethoxyethane, trimethoxymethane, 1,1-dichloro-2,2-difluoroethylmethylether, 2,2-diethoxypropane, isobutyl-tert-butylether, sec-butyl-tert-butylether, 1,1-diethoxypropane, 2-methoxyethanol, 2-chloro-1,1-dimethoxyethane, methoxycyclohexane, ethoxyethanol, di-n-butylether, 1-ethoxy-hexane, 1-methoxy-2-acetoxypropane, 1,1,1-triethoxyethane.

De préférence, les aldéhydes sont sélectionnés parmi le groupe consistant en Acetaldehyde, ethanedial, isobutanal, methylglyoxal, 2-methylbutanal, 2,6-dimethyl-5-heptenal, hexanal.

De préférence, les nitriles sont sélectionnés parmi le groupe consistant en Acetonitrile, propionitrile, butyronitrile, valeronitrile, (methyleneamino)acetonitrile.

De préférence, le carbonate est diethylcarbonate.

De préférence, l'amide est éthanethioamide.

De préférence, les thioalkyles sont sélectionnés parmi le groupe consistant en éthanethiol, dimethylsulfide, 2-propanetiol, 4-methoxy-2-methyl-2-butanethiol, tert-butylthiol, 1-propanethiol, thiophene, 2-butanethiol, 2-methyl-1-propanethiol, diethylsulfide, butanethiol, 3, mercapto-1,2-propanediol, tetrahydrothiophene, 1-pentanethiol.

De préférence, les hétérocycles sont choisis parmi le groupe consistant en furane, 1,2-epoxypropane, tetrahydrofurane, dioxane, 1,3-dioxane, piperidine, 1,3,5-trioxane, n-methylmorpholine, 2-methylpyrazine, 1-methylpiperazine, n-ethyl-morpholine, 2,6-dimethylmorpholine, 3-furfural.

Ledit agent d'extraction organique peut être Chloroethane, 3-chloro-1,1,1,2,2,3-hexafluoropropane, 1,1,1,2,2,3,3,4,4-nonafluorobutane, 2-chloro-1,1,1,3,3,3-hexafluoropropane, 1,1,1,3,3-pentafluoropropane, ethylamine, bromofluoromethane, 1-bromo-1,2-difluoroethylene, 1-chloro-1,1,2,3,3,3-hexafluoropropane, 1-chloro-1,1,2,2-tetrafluoropropane, acetaldehyde, 1-chloro-1,1,2,2,3,3-hexafluoropropane, 1,1,1-trifluoro-2-propanone, 2-chloro-1,1,1,2-tetrafluoropropane, 1,1,1,2,3-pentafluoropropane, trichlorofluoromethane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 2,2,2-trifluoroethylmethylether, 1,1-dichloroethylene, furane, methylformate, isopropylamine, 1,1,2,2-tetrafluoroethylmethylether, 1,1,1,2,3,4,4,4-octafluorobutane, 2-methoxy-1-propene, heptafluoro-1-methoxypropane, diethylether, 1,2-epoxypropane, ethanethiol, 2-chloro-2-fluoropropane, ethoxy-ethene, 1-chloro-2,2-difluoroethane, ethylmethylamine, dimethylsulfide, 2-chloropropane, bromoethane, dichloromethane, dimethoxymethane, 2-chloro-1,1,1,3,3-pentafluoropropane, 1-chloro-1,2,2-trifluoropropane, iodomethane, 2,2-dichloro-1,1,1,3,3-pentafluoropropane, 2-amino-2-methylpropane, methylcyclopropylether, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, n-propylamine, 1,1,2-trichloro-1,2,2-trifluoroethane, 2,2, dimethylbutane, isopropylmethylamine, ethanedial, 1,2-dichloro-1,1,3,3,3-pentafluoropropane, 1-chloro-1,2,2,3-tetrafluoropropane, perfluorocyclohexane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 2-propanethiol, 1,3-dichloro-1,1,2,3,3-pentafluoropropane, 1,2-dichloro-3,3,3-trifluoropropene, 1,3-dichloro-1,1,2,2,3-pentafluoropropane, 1,1,1,2,2,3,4,5,5,5-decafluoropentane, 2-ethoxy-propane, 2,2-dichloro-1,1,1,3-tetrafluoropropane, 1-chloro-2,2-difluoropropane, methyl-t, butylether, diethylamine, propanone, perfluoro-n-hexane, methylacetate, 1,1-dichloroethane, ethyl1,2,2-tetrafluoroethylether, 4-methoxy-2-methyl-2-butanethiol, 2-bromopropane, chloromethoxymethane, 1,2-dichloro-1,2,3,3,4,4-hexafluorocyclobutane, 1,1-dichloro-2,2-difluoroethane, 2,2-dichloro-1,1,3,3-tetrafluoropropane, difluorodiethylsilane, 2-butanamine, 2,3-dichloro-1,1,1,2-tetrafluoropropane, n-methylpropylamine, 3-methylpentane, 1,1-dichloro-1,2,2,3-tetrafluoropropane, tert-butylthiol, isobutanal, methanol, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, chlorobromomethane, 2-chlorobutane, isopropylformate, diisopropylether, 1,3-dichloro-1,1,3,3-tetrafluoropropane, hexane, 1,3-dichloro-1,2,2,3-tetrafluoropropane, 1,2-dichloro-1,1,2,3-tetrafluoropropane, 3-bromopropene, 2,3-dichloro-1,1,1,3-tetrafluoropropane, 1,1-dichloro-2-fluoroethane, 1-bromopropane, 1,1-difluoro-1,2,2-trichloroethane, 1,1,2-trichloro-1,2-difluoroethane, methylglyoxal, iodoethane, 2-ethoxy-2-methyl-propane, 2-bromo-2-methylpropane, 1,2-dichloro-1-fluoroethane, 1,1,1-trichloroethane, 2,2,2-trifluoroethanol, 1-chloro-3-fluoropropane, 1,1,1-trifluoro-2-propanol, 2,3-dichloro-1,1,1-trifluoropropane, perfluoromethylcyclohexane, ethylacetate, 1-butylamine, 1-chlorobutane, ethanol, butanone, 2,4-dimethylpentane, cyclohexane, n-propylformate, 2-ethoxy-butane, acetonitrile, pentafluoro-1-propanol, 2-propanol, tert-butanol, perfluoroheptane, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1,2-dichloroethane, 1-ethoxy-2-methylpropane, diisopropylamine, thiophene, 2-butanethiol, 1,1,1-trichloro-2,2,3,3-tetrafluoropropane, 1,2-dimethoxyethane, 3-methyl-2-butanamine, 1,1,3-trichloro-1,2,2,3-tetrafluoropropane, 1,3-dichloro-1,2,2-trifluoropropane, diethoxymethane, 1,1-dichloropropane, 2-methyl-1-propanethiol, 1,2-dichloro-2-fluoropropane, isopropylacetate, 2-iodopropane, di-n-propylether, 3-pentylamine, n-methylbutylamine, 2-bromobutane, 2,2-difluorotetrachloroethane, diethylsulfide, 1-ethoxybutane, 1,1,2,2-tetrachloro-1,2-difluoroethane, 1-fluorohexane, 1-methoxy-2-propanamine, 1,3-dichloro-1,2,3-trifluoropropane, 2,3-dichloro-1-propene, 2-methoxyethanamine, 2,2-difluoroethanol, 2-methylbutanal, 1,2-dichloropropane, propanol, tert-butylacetate, propionitrile, 3-chloropentane, trichloroacetaldehyde, 2-allyloxyethanol, butanethiol, isoamylchloride, 1-methoxy-pentane, ethylpropionate, 2-butanol, 1,2-dimethoxypropane, isopropyl-isobutylether, 1,1,1-trichloro-2,2,3-trifluoropropane, methylcyclohexane, 1-chloro-4-fluorobutane, 1-bromo-3-fluoropropane, 2,4,4-trimethyl-1-pentene, dioxane, 1-bromobutane, 3-pentanone, 1,1,2-trichloro-2-fluoroethane, 1,1-diethoxyethane, 2-pentanone, 2-methyl-2-butanol, 1-iodopropane, 2-methoxy-lpropanamine, 1,1,3-trichloro-1,2,2-trifluoropropane, 1,1,3-trichloro-2,2,3-trifluoropropane, trimethoxymethane, cis-1,3-dichloropropene, 2,2-dichlorobutane, n-pentylamine, 1,1-dichloro-2,2-difluoroethylmethylether, 2,2,4-trimethyl-2-pentene, perfluorooctane, 1,1,1,2-tetrachloro-2-fluoroethane, 1,3-dioxane, 3,3-dimethyl-2-butanone, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, 1-methylcyclohexene, toluene, trans-1,3-dichloropropene, sec-butylacetate, 2-fluorotoluene, 2,2-dimethyl-1-propanol, 1,1,1-trichloro-3-fluoropropane, n-methyl-1,2-ethanediamine, 2,2-diethoxypropane, 1,3,5-trioxane, 3,3,3-trichloro-1-propene, 1-chloro-3,3-dimethylbutane, pyridine, 2,3-dimethylhexane, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,1,3,3-tetrachloro-1,2,2-trifluoropropane, 1,2-propanediamine, 2,6-dimethyl-5-heptenal, perfluorobutanoicacid, 1,1,1,3-tetrachloro-2,2,3-trifluoropropane, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 1,2-dichlorobutane, 4,4,4-trifluorobutanol, 2-ethylbutylamine, perfluorononane, octane, diethylcarbonate, n-butylacetate, 1-pentanethiol, 1,2,2,3-tetrachloro-3,3-difluoropropane, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxyl-propanol, 1,2-dichloro-2-butene, 1-iodobutane, 1-ethoxy-2-propanol, hexanal, 4-methyl-2-pentanol, 1,2-dibromoethane, chlorobenzene, ethylcyclohexane, perfluorooctylbromide, bromoaceticacidmethylester, 1,1,2-trichloropropane, 1,2-octanediol, 4-methyl-2-hexanamine, hexylamine, 2-chloro-1-propanol, , 2-(dimethylamino)-ethanol, 1,3-dichlorobutane, cyclohexylamine, n-ethyl-2-dimethylaminoethylamine, ethoxyethanol, 3-hexanol, 2-hexanol, ethylbenzene, 2-methylpyrazine, 2-ethoxy-1-propanol, 1-pentanol, 1-methylpiperazine, n-ethyl-morpholine, 1,4-dimethylbenzene, 1,3-dimethylbenzene, 1,3-propanediamine, di-n-butylether, 3,3,4,4,5,5,6,6-octafluoro-1-pentanol,valeronitrile, (methyleneamino)acetonitrile, 1,2-dibromopropane, 2-heptanamine, 1,2,3-trichloropropene, 1,2,3-trimethylcyclohexane, 2,3-dimethylbutanol, 1-ethoxy-hexane, 1-chloro-3-bromopropane, perfluoro-n-decane, 3-furfural, n,n-diethylethylenediamine, 2,6-dimethylpyridine, 1,1,3,3-tetrachloro-1-fluoropropane, 4-methyl-2-hexanone, 1,2-dimethylbenzene, 1,1,2,2,3-pentchloro-3,3-difluoropropane, 1-methoxy-2-acetoxypropane, 1,1,1-triethoxyethane, styrene, 4-methylpyridine, n,n'-diethyl-1,2-ethanediamine, 2,6-dimethylmorpholine, 2-ethyl-1-butanol, 1,2-dichloropentane, 2-methyl-1-pentanol, methylhexanoate, 2-propoxyethanol, dimethylethanolamine, 1-propoxy-2-propanol. Avantageusement, ledit agent d'extraction organique peut être Chloroethane, 3-chloro-1,1,1,2,2,3-hexafluoropropane, 1,1,1,2,2,3,3,4,4-nonafluorobutane, 2-chloro, 1,1,1,3,3,3-hexafluoropropane, 1,1,1,3,3-pentafluoropropane, ethylamine, bromofluoromethane, 1-bromo-1,2-difluoroethylene, 1-chloro-1,1,2,3,3,3-hexafluoropropane, 1-chloro-1,1,2,2-tetrafluoropropane, acetaldehyde, 1-chloro-1,1,2,2,3,3-hexafluoropropane, 1,1,1-trifluoro-2-propanone, 2-chloro-1,1,1,2-tetrafluoropropane, 1,1,1,2,3-pentafluoropropane, 1-chloro-1,1-difluoropropane, 1,1,1-trifluoro-2-bromoethane, 1,1-dichloro-2,2,2-trifluoroethane, 1-chloro 2,2,3,3,3-pentafluoropropane, 1,2-dichloro-1,2-trifluoroethane, 2,2,2-trifluoroethylmethylether, furane, methylformate, isopropylamine, 1,1,2,2-tetrafluoroethylmethylether, 1,1,1,2,3,4,4,4, octafluorobutane, 2-methoxy-1-propene, heptafluoro-1-methoxypropane, diethylether, 1,2-epoxypropane, ethanethiol, 2-chloro-2-fluoropropane, ethoxy-ethene, 1-chloro-2,2-difluoroethane, ethylmethylamine, dimethylsulfide, 2-chloropropane, bromoethane, dimethoxymethane, 2-chloro-1,1,1,3,3-pentafluoropropane, 1-chloro-1,2,2-trifluoropropane, 2,2-dichloro-1,1,1,3,3-pentafluoropropane, 2-amino-2-methylpropane, methylcyclopropylether, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, n-propylamine, 1,1,2-trichloro-1,2,2-trifluoroethane, isopropylmethylamine, ethanedial, 1,2-dichloro-1,1,3,3,3-pentafluoropropane, 1-chloro-1,2,2,3-tetrafluoropropane, perfluorocyclohexane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 2-propanethiol, 1,2-dichloro-3,3,3-trifluoropropene, 1,1,1,2,2,3,4,5,5,5-decafluoropentane, 2-ethoxy-propane, 2,2-dichloro-1,1,1,3-tetrafluoropropane, 1-chloro-2,2-difluoropropane, methyl-t-butylether" diethylamine, propanone, perfluoro-n-hexane, methylacetate, ethyl1,1,2,2-tetrafluoroethylether, 4-methoxy-2-methyl-2-butanethiol, 2-bromopropane, 1,2-dichloro-1,2,3,3,4,4-hexafluorocyclobutane, 1,1-dichloro-2,2-difluoroethane, 2,2-dichloro-1,1,3,3-tetrafluoropropane, difluorodiethylsilane, 2-butanamine, 2,3-dichloro-1,1,1,2-tetrafluoropropane, n-methylpropylamine, 1,1-dichloro-1,2,2,3-tetrafluoropropane, tert-butylthiol, isobutanal, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, 2-chlorobutane, isopropylformate, diisopropylether, 1,3-dichloro-1,2,2,3-tetrafluoropropane, 1,2-dichloro-1,1,2,3-tetrafluoropropane, 2,3-dichloro-1,1,1,3-tetrafluoropropane, 1-bromopropane, 1,1-difluoro-1,2,2-trichloroethane, 1,1,2-trichloro-1,2-difluoroethane, methylglyoxal, 2-ethoxy-2-methyl-propane, 2-bromo-2-methylpropane, 1-chloro-3-fluoropropane, 1,1,1-trifluoro-2-propanol, 2,3-dichloro-1,1,1-trifluoropropane, perfluoromethylcyclohexane, ethylacetate, 1-butylamine, 1-chlorobutane, butanone, n-propylformate, 2-ethoxy-butane, pentafluoro-1-propanol, 2-propanol, tert-butanol, perfluoroheptane, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1-ethoxy-2-methylpropane, diisopropylamine, 2-butanethiol, 1,1,1-trichloro-2,2,3,3-tetrafluoropropane, 1,2-dimethoxyethane, 3-methyl-2-butanamine, 1,1,3-trichloro-1,2,2,3-tetrafluoropropane, 1,3-dichloro-1,2,2-trifluoropropane, diethoxymethane, 2-methyl-1-propanethiol, isopropylacetate, 2-iodopropane, di-n-propylether, 3-pentylamine, n-methylbutylamine, 2-bromobutane, diethylsulfide, 1-ethoxybutane, 1-fluorohexane,, 1-methoxy-2-propanamine, 1,3-dichloro-1,2,3-trifluoropropane, 2-methoxyethanamine, 2, methylbutanal, propanol, tert-butylacetate, propionitrile, 3-chloropentane, 2-allyloxyethanol, butanethiol, isoamylchloride, 1-methoxypentane, ethylpropionate, 2-butanol, 1,2-dimethoxypropane, isopropyl-isobutyl-ether, 1,1,1-trichloro-2,2,3-trifluoropropane, 1-chloro-4-fluorobutane, 1-bromo-3-fluoropropane, 2,4,4-trimethyl-1-pentene, dioxane, 1-bromobutane, 3-pentanone, 1,1-diethoxyethane, 2-pentanone, 2-methyl-2-butanol, 2-methoxy-lpropanamine, 1,1,3-trichloro-1,2,2-trifluoropropane, 1,1,3-trichloro-2,2,3-trifluoropropane, trimethoxymethane, n-pentylamine, 1,1-dichloro-2,2-difluoroethylmethylether, 2,2,4-trimethyl-2-pentene, perfluorooctane, 1,3-dioxane, 3,3-dimethyl-2-butanone, piperidine, isobutanol, 2-bromo-2-methylbutane, dipropylamine, 2,2,3,3-tetraflouro-1-propanol, 2-ethoxyethanamine, triethylfluorosilane, 1-methylcyclohexene, toluene, sec-butylacetate, 2-fluorotoluene, 2,2-dimethyl-1-propanol, n-methyl, 1,2-ethanediamine, 2,2-diethoxypropane, 1,3,5-trioxane, 1-chloro-3,3-dimethylbutane, pyridine, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, sec-butyl-tert-butylether, 1-methoxy2-propanol, 1,1,3,3-tetrachloro-1,2,2-trifluoropropane, 1,2-propanediamine, 2,6-dimethyl-5-heptenal, perfluorobutanoicacid, 1,1,1,3-tetrachloro-2,2,3-trifluoropropane, 1-bromo-3-methylbutane, 1-(dimethylamino)-2-propanol, tetrahydrothiophene, 3-methyl-3-pentanol, 1,1-diethoxypropane, 2-methoxyethanol, 4,4,4-trifluorobutanol, 2-ethylbutylamine, perfluorononane, diethylcarbonate, n-butylacetate, 1-pentanethiol, 2-chloro-1,1-dimethoxyethane, 2-hexanone, n-ethylethylenediamine, 5-hexen-2-one, 1,1-diethoxy-n,n-dimethylmethanamine, 2-methylpyridine, 1-bromopentane, 2-methoxyl-propanol, 1-ethoxy-2-propanol, hexanal, 4-methyl-2-pentanol, perfluorooctylbromide, 1,2-octanediol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 3-hexanol, 2-hexanol, ethylbenzene, 2-methylpyrazine, 2-ethoxy-1-propanol, 1-pentanol, 1-methylpiperazine, n-ethyl-morpholine, 1,4-dimethylbenzene, 1,3-dimethylbenzene, 1,3-propanediamine, di-n-butylether, 3,3,4,4,5,5,6,6-octafluoro-1-pentanol, valeronitrile, (methyleneamino)acetonitrile, 2-heptanamine, 2,3, dimethylbutanol, 1-ethoxy-hexane, perfluoro-n-decane, 3-furfural, n,n-diethylethylenediamine, 2,6-dimethylpyridine, 4-methyl-2-hexanone, 1,2-dimethylbenzene, 1-methoxy-2-acetoxypropane, 1,1,1-triethoxyethane, styrene, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, dimethylethanolamine, 1-propoxy-2-propanol.

De préférence, ledit agent d'extraction organique peut être éthylamine, acetaldehyde, 1,1,1,3,3-pentafluoropropane, 1,1,1-trifluoro-2-propanone, 1,1,1,2,3-pentafluoropropane, 2,2,2-trifluoroethylmethylether, methylformate, isopropylamine, 1,1,2,2-tetrafluoroethylmethylether, 2-methoxy-1-propene, diethylether, 1,2-epoxypropane, ethoxy-ethene, ethylmethylamine, dimethoxymethane, 1-chloro-1,2,2-trifluoropropane, 2-amino-2-methylpropane, methylcyclopropylether, 3-chloro-1,1,1-trifluoropropane, n-propylamine, isopropylmethylamine, ethanedial, 1-chloro-1,2,2,3-tetrafluoropropane, 3-chloro-1,1,1,3-tetrafluoropropane, 2-chloro-1,1,1,3-tetrafluoropropane, 2-ethoxy-propane, methyl-t-butylether, diethylamine, propanone, methylacetate, ethyl1,1,2,2-tetrafluoroethylether, 4-methoxy-2-methyl-2-butanethiol, difluorodiethylsilane, 2-butanamine, n-methylpropylamine, isobutanal, tetrahydrofurane, 1,3-dichloro-1,1,2,2-tetrafluoropropane, isopropylformate, diisopropylether, methylglyoxal, 2-ethoxy-2-methyl-propane, 2,3-dichloro-1,1,1-trifluoropropane, ethylacetate, 1-butylamine, butanone, n-propylformate, 2-ethoxy-butane, 1,3-dichloro-1,1,2-trifluoropropane, 1-methoxy-2-methyl-butane, 1,1-dichloro-2,2,3-trifluoropropane, 2,2-dimethoxypropane, 1-ethoxy-2-methylpropane, diisopropylamine, 1,2-dimethoxyethane, 3-methyl-2-butanamine, 1,3-dichloro-1,2,2-trifluoropropane, diethoxymethane, isopropylacetate, di-n-propylether, 3-pentylamine, n-methylbutylamine, diethylsulfide, 1-ethoxybutane, 1-fluorohexane, 1-methoxy-2-propanamine, 1,3-dichloro-1,2,3-trifluoropropane, 2-methoxyethanamine, 2-methylbutanal, tert-butylacetate, propionitrile, 2-allyloxyethanol, 1-methoxy-pentane, ethylpropionate, 1,2-dimethoxypropane, isopropyl-isobutyl-ether, dioxane, 3-pentanone, 1,1-diethoxyethane, 2-pentanone, 2-methoxy-1propanamine, trimethoxymethane, n-pentylamine, 1,3-dioxane, 3,3-dimethyl-2-butanone, piperidine, dipropylamine, 2-ethoxyethanamine, triethylfluorosilane, sec-butylacetate, n-methyl-1,2-ethanediamine, 2,2-diethoxypropane, pyridine, n-methylmorpholine, 4-methyl-2-pentanone, 1,2-diaminoethane, isobutyl-tert-butylether, butyronitrile, sec-butyl-tert-butylether, 1-methoxy2-propanol, 1,2-propanediamine, 2,6-dimethyl-5-heptenal, perfluorobutanoicacid, 1-(dimethylamino)-2-propanol, 3-methyl-3-pentanol, 1,1-diethoxypropane, 2-ethylbutylamine, diethylcarbonate, n-butylacetate, 2-hexanone, n-ethylethylenediamine, 5-hexen-2-one, 1,1-diethoxy-n,n-dimethylmethanamine, 2-methylpyridine, 2-methoxyl-propanol, 1-ethoxy-2-propanol, hexanal, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-methylpyrazine, 2-ethoxy-1-propanol, 1-methylpiperazine, n-ethyl-morpholine, 1,3-propanediamine, di-n-butylether, 3,3,4,4,5,5,6,6-octafluoro-1-pentanol, valeronitrile, 2-heptanamine, 1-ethoxy-hexane, n,n-diethylethylenediamine, 2,6-dimethylpyridine, 4-methyl-2-hexanone, 1-methoxy-2-acetoxypropane, 1,1,1-triethoxyethane, 4-methylpyridine, n,n'-diethyl-1,2-ethanediamine, 2,6-dimethylmorpholine, methylhexanoate, 2-propoxyethanol, 1-propoxy-2-propanol.

En particulier, ledit agent d'extraction organique peut être éthylamine, 1,1,1-trifluoro-2-propanone, methylformate, isopropylamine, 2-methoxy-1-propene, diethylether, 1,2-epoxypropane, ethoxy-ethene, ethylmethylamine, dimethoxymethane, 2-amino-2-methylpropane, methylcyclopropylether, n-propylamine, isopropylmethylamine, 2-ethoxy-propane, methyl-t-butylether, diethylamine, propanone, methylacetate, 2-butanamine, n-methylpropylamine, isobutanal, tetrahydrofurane, isopropylformate, diisopropylether, 2-ethoxy-2-methyl-propane, ethylacetate, 1-butylamine, butanone, n-propylformate, 2-ethoxybutane, 1-methoxy-2-methyl-butane, 2,2-dimethoxypropane, 1-ethoxy-2-methylpropane, 1,2-dimethoxyethane, 3-methyl-2-butanamine, diethoxymethane, isopropylacetate, di-n-propylether, 3-pentylamine, n, methylbutylamine, 1-ethoxybutane, 1-methoxy-2-propanamine, 2-methoxyethanamine, 2-methylbutanal, tert-butylacetate, 1-methoxy-pentane, ethylpropionate, 1,2-dimethoxypropane, dioxane, 3-pentanone, 1,1-diethoxyethane, 2-pentanone, 2-methoxy-lpropanamine, trimethoxymethane, n-pentylamine, 1,3-dioxane, 3,3-dimethyl-2-butanone, piperidine, 2-ethoxyethanamine, sec-butylacetate, n-methyl-1,2-ethanediamine, 2,2-diethoxypropane, 4-methyl-2-pentanone, 1,2-diaminoethane, butyronitrile, 1-methoxy2-propanol, 1,2-propanediamine, 2,6-dimethyl-5-heptenal, 1-(dimethylamino)-2-propanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxyl-propanol, 1-ethoxy-2-propanol, hexanal, 4-methyl-2-hexanamine, hexylamine, methoxycyclohexane, 2-(dimethylamino)-ethanol, cyclohexylamine, n-ethyl-2-dimethylaminoethylamine, 2-methylpyrazine, 2-ethoxy-1-propanol, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1-methoxy-2-acetoxypropane, 1,1,1-triethoxyethane, 4-methylpyridine, n,n'-diethyl-1,2-ethanediamine, 2,6-dimethylmorpholine, methylhexanoate, 2-propoxyethanol, 1-propoxy-2-propanol.

Plus particulièrement, ledit agent d'extraction organique peut être éthylamine, isopropylamine, diethylether, ethoxy-ethene, dimethoxymethane, n-propylamine, methyl-t-butylether, diethylamine, propanone, methylacetate, isobutanal, tetrahydrofurane, isopropylformate, diisopropylether, 2-ethoxy-2-methyl-propane, ethylacetate, butanone, diethoxymethane, isopropylacetate, 3-pentylamine, 2-methoxyethanamine, tert-butylacetate, dioxane, 3-pentanone, 1,1-diethoxyethane, 2-pentanone, trimethoxymethane, n-pentylamine, 1,3-dioxane, 3,3-dimethyl-2-butanone, sec-butylacetate, 4-methyl-2-pentanone, 1,2-diaminoethane, 1-methoxy2-propanol, diethylcarbonate, n-butylacetate, 1-ethoxy-2-propanol, hexanal.

De manière privilégiée, ledit agent d'extraction organique peut être choisi parmi éthylamine, isopropylamine, diethylether, dimethoxymethane, n-propylamine, , diethylamine, , diisopropylether, 2-ethoxy-2-methyl-propane, butanone, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal. De manière plus privilégiée, ledit agent d'extraction organique peut être choisi parmi éthylamine, isopropylamine, diethylether, dimethoxymethane, n-propylamine, diethylamine, diisopropylether, 2-ethoxy-2-methyl-propane, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal.

L'agent d'extraction organique à utiliser peut être sélectionné en fonction des composés présents dans ladite première composition. Ainsi, l'agent d'extraction organique peut être sélectionné en fonction du facteur de séparation et de la capacité d'absorption établi pour une composition particulière. Outre ces deux critères, le choix de l'agent d'extraction organique peut se baser optionnellement sur d'autres critères commerciaux ou environnementaux, tels que par exemple le coût de l'agent d'extraction organique, sa disponibilité sur le marché, ses propriétés toxiques ou inflammable. De plus, selon un mode de réalisation particulier, afin d'optimiser le fonctionnement des colonnes de distillation utilisées à l'étape b) et c) du présent procédé de purification du 2,3,3,3-tetrafluoro-1-propene, le point d'ébullition de l'agent d'extraction organique peut être de 10°C à 200°C, avantageusement de 10°C à 190°C, de préférence de 10°C à 180°C, en particulier de 10°C à 170°C, plus particulièrement de 10°C à 160°C, et de manière privilégiée de 10°C à 150°C.

Selon un mode de réalisation préféré, ledit agent d'extraction organique a un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle

$\gamma_{1,S}$ représente le coefficient d'activité du 2,3,3,3-tétrafluoro-1-propène dans ledit agent d'extraction organique à dilution infinie,
P1 représente la pression de vapeur saturante du 2,3,3,3-tétrafluoro-1-propène,
$y_{2,s}$ représente le coefficient d'activité dudit au moins un des composés consistant en 1,1-difluoroéthane (152a), chloropentafluoroéthane (115), trans-1,3,3,3-tetrafluoro-1-propene (1234ze-E), dans ledit agent d'extraction organique à dilution infinie, avantageusement le coefficient d'activité de trans-1,3,3,3-tetrafluoro-1-propene (1234ze-E), P2 représente la pression de vapeur saturante de dudit au moins un des composés consistant en 1,1-difluoroéthane (152a), chloropentafluoroéthane (115), trans-1,3,3,3-tetrafluoro-1-propene (1234ze-E), avantageusement la pression de vapeur saturante de trans-1,3,3,3-tetrafluoro-1-propene (1234ze-E).

Avantageusement, le facteur de séparation $S_{1,2}$ est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

Selon un mode de réalisation préféré, ledit agent d'extraction organique a une capacité d'absorption $C_{2,S}$ supérieure ou égale à 0,20, ladite capacité d'absorption étant calculé par la formule $C_{2,S} = 1/(\gamma_{2,S})$ dans laquelle $\gamma_{2,S}$ représente le coefficient d'activité dudit au moins un des composés consistant en 1,1-difluoroéthane (152a), chloropentafluoroéthane (115), trans-1,3,3,3-tetrafluoro-1-propène (1234ze-E) dans ledit agent d'extraction organique à dilution infinie, avantageusement le coefficient d'activité de 1,3,3,3-tetrafluoro-1-propène (1234ze-E).

Avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 1,0.

Selon un mode de réalisation préféré, ledit agent d'extraction organique peut avoir un facteur de séparation $S_{1,2}$ supérieur ou égal à 1,1, avantageusement supérieur ou égal à 1,2, de préférence supérieur ou égal à 1,4, plus préférentiellement supérieur ou égal à 1,6, en particulier supérieur ou égal à 1,8 et plus particulièrement supérieur ou égal à 2,0; et une capacité d'absorption $C_{2,S}$ supérieure ou égale à 0,20, avantageusement supérieure ou égale à 0,40, de préférence supérieure ou égale à 0,60, plus préférentiellement supérieure ou égale à 0,80, en particulier supérieure ou égale à 1,0.

Ladite première composition peut être une composition azéotropique ou quasi-azéotropique comprenant 2,3,3,3-tetrafluoro-1-propène, et ledit au moins un des composés sélectionné parmi le groupe consistant en 1,1-difluoroéthane (152a), chloropentafluoroéthane (115), trans-1,3,3,3-tetrafluoro-1-propène (1234ze-E). Avantageusement, ladite première composition peut être une composition azéotropique ou quasi-azéotropique comprenant 2,3,3,3-tetrafluoro-1-propène, trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115).

Ladite première composition peut être une composition azéotropique ou quasi-azéotropique comprenant 2,3,3,3-tetrafluoro-1-propène et au moins deux ou l'ensemble des composés sélectionné parmi le groupe consistant en 1,1-difluoroéthane (152a), chloropentafluoroéthane (115), trans-1,3,3,3-tetrafluoro-1-propene (1234ze-E). Avantageusement, ladite première composition peut être une composition azéotropique ou quasi-azéotropique comprenant 2,3,3,3-tetrafluoro-1-propène, trans-1,3,3,3-tetrafluoro-1-propene (1234ze-E) et au moins un ou les deux composés sélectionnés parmi le groupe consistant en 1,1-difluoroéthane (152a), chloropentafluoroéthane (115).

En fonction du ou des composés à éliminer dans ladite première composition, ledit facteur de séparation et ladite capacité d'absorption pourront être calculés pour un couple binaire particulier consistant en 2,3,3,3-tetrafluoro-1-propène et un des composés choisi parmi le groupe consistant en 1,1-difluoroéthane (152a), chloropentafluoroéthane (115), trans-1,3,3,3-tetrafluoro-1-propene (1234ze-E). Ainsi, pour sélectionner ledit agent d'extraction organique apte à être utilisé dans l'étape b) de distillation extractive, le facteur de séparation et la capacité d'absorption peuvent être calculés par exemple pour un couple binaire 2,3,3,3-tetrafluoro-1-propène et trans-1,3,3,3-tetrafluoro-1-propene (1234ze-E) si ladite première composition comprend ces deux composés. Le facteur de séparation $S_{1,2}$ permet de déterminer la capacité d'un agent d'extraction organique à séparer deux ou plusieurs composés. La capacité d'absorption $C_{2,S}$ permet de déterminer la quantité de solvant à utiliser pour obtenir la séparation entre les composés considérés. Pour l'ensemble des premières compositions détaillées ci-dessous, ledit agent d'extraction organique peut avoir un facteur de séparation $S_{1,2}$ supérieur ou égal à 1,1, avantageusement supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0; et/ou ledit agent d'extraction organique peut avoir une capacité d'absorption $C_{2,S}$ supérieure ou égale à 0,20, avantageusement supérieure ou égale à 0,40, de préférence supérieure ou égale à 0,60, plus préférentiellement supérieure ou égale à 0,80, en particulier supérieure ou égale à 1,0.

En particulier, ladite première composition peut être une composition azéotropique ou quasi-azéotropique comprenant 2,3,3,3-tetrafluoro-1-propène et trans-1,3,3,3-tetrafluoro-1-propene (1234ze-E). La teneur en chacun des composés dans cette composition particulière est telle qu'exprimée ci-dessus en référence aux teneurs individuelles de chacun des composés. Ainsi, la seconde composition peut comprendre 2,3,3,3-tetrafluoro-1-propène, trans-1,3,3,3-tetrafluoro-1-propene (1234ze-E) et ledit agent d'extraction organique. Ledit agent d'extraction organique peut avoir un facteur de séparation $S_{1,2}$ supérieur ou égal à 1,1, ledit facteur de séparation étant calculé par la formule $S_{1,2} = (\gamma_{1,S}*P1)/(\gamma_{2,S}*P2)$ dans laquelle $\gamma_{1,S}$ représente le coefficient d'activité du 2,3,3,3-tetrafluoro-1-propène par rapport audit agent d'extraction organique, P1 représente la pression de vapeur saturante du 2,3,3,3-tetrafluoro-1-propène, $\gamma_{2,S}$ représente le coefficient d'activité du trans-1,3,3,3-tetrafluoro-1-propene dans ledit agent d'extraction organique à dilution infinie, P2 représente la pression de vapeur saturante du trans-1,3,3,3-tetrafluoro-1-propene. Ledit agent d'extraction organique peut avoir une capacité d'absorption $C_{2,S}$ supérieure ou égale à 0,2, ladite capacité d'absorption étant calculé par la formule $C_{2,S} = 1/(\gamma_{2,S})$ dans laquelle $\gamma_{2,S}$ représente le coefficient d'activité dans ledit agent d'extraction organique à dilution infinie. Ainsi, pour séparer une première composition azéotropique ou quasi-azéotropique comprenant 2,3,3,3-tetrafluoro-1-propène et trans-1,3,3,3-tetrafluoro-1-propene (1234ze-E), un agent d'extraction organique ayant un facteur de séparation $S_{1,2}$ supérieur ou égal à 1,1 et une capacité d'absorption $C_{2,S}$ supérieure ou égale à 0,2 peut être utilisé ; ledit agent d'extraction organique peut ainsi être chloroéthane, 3-chloro-1,1,1,2,2,3-hexafluoropropane, 1,1,1,2,2,3,3,4,4-nonafluorobutane, 2-chloro-1,1,1,3,3,3-hexafluoropropane, 1,1,1,3,3-pentafluoropropane, ethylamine, bromofluoromethane, 1-bromo-1,2-difluoroethylene, 1-chloro-1,1,2,3,3,3-hexafluoropropane, 1-chloro-1,1,2,2-tetrafluoropropane, acetaldehyde, 1-chloro-1,1,2,2,3,3-hexafluoropropane, 1,1,1-trifluoro-2-propanone, 2-chloro-1,1,1,2-tetrafluoropropane, 1,1,1,2,3-pentafluoropropane, trichlorofluoromethane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 2,2,2-trifluoroethylmethylether, 1,1-dichloroethylene, furane, methylformate, isopropylamine, 1,1,2,2-tetrafluoroethylmethylether, 1,1,1,2,3,4,4,4-octafluorobutane, 2-methoxy-1-propene, heptafluoro-1-methoxypropane, diethylether, 1,2-epoxypropane, ethanethiol, 2-chloro-2-fluoropropane, ethoxy-ethene, 1-chloro-2,2-difluoroethane, ethylmethylamine, dimethylsulfide, 2-chloropropane, bromoethane, dichloromethane, dimethoxymethane, 2-chloro-1,1,1,3,3-pentafluoropropane, 1-chloro-1,2,2-trifluoropropane, iodomethane, 2,2-dichloro-1,1,1,3,3-pentafluoropropane, 2-amino-2-methylpropane, methylcyclopropylether, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, n-propylamine, 1,1,2-trichloro-1,2,2-trifluoroethane, 2,2, dimethylbutane, isopropylmethylamine, ethanedial, 1,2-dichloro-1,1,3,3,3-pentafluoropropane, 1-chloro-1,2,2,3-tetrafluoropropane, perfluorocyclohexane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 2-propanethiol, 1,3-dichloro-1,1,2,3,3-pentafluoropropane, 1,2-dichloro-3,3,3-trifluoropropene, 1,3-dichloro-1,1,2,2,3-pentafluoropropane, 1,1,1,2,2,3,4,5,5,5-decafluoropentane, 2-ethoxy-propane, 2,2-dichloro-1,1,1,3-tetrafluoropropane, 1-chloro-2,2-difluoropropane, methyl-t, butylether, diethylamine, propanone, perfluoro-n-hexane, methylacetate, 1,1-dichloroethane, ethyl1,1,2,2-tetrafluoroethylether, 4-methoxy-2-methyl-2-butanethiol, 2-bromopropane, chloromethoxymethane, 1,2-dichloro-1,2,3,3,4,4-hexafluorocyclobutane, 1,1-dichloro-2,2-difluoroethane, 2,2-dichloro-1,1,3,3-tetrafluoropropane, difluorodiethylsilane, 2-butanamine, 2,3-dichloro-1,1,1,2-tetrafluoropropane, n-methylpropylamine, 3-methylpentane, 1,1-dichloro-1,2,2,3-tetrafluoropropane, tert-butylthiol, isobutanal, methanol, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, chlorobromomethane, 2-chlorobutane, isopropylformate, diisopropylether, 1,3-dichloro-1,1,3,3-tetrafluoropropane, hexane, 1,3-dichloro-1,2,2,3-tetrafluoropropane, 1,2-dichloro-1,1,2,3-tetrafluoropropane, 3-bromopropene, 2,3-dichloro-1,1,1,3-tetrafluoropropane, 1,1-dichloro-2-fluoroethane, 1-bromopropane, 1,1-difluoro-1,2,2-trichloroethane, 1,1,2-trichloro-1,2-difluoroethane, methylglyoxal, iodoethane, 2-ethoxy-2-methyl-propane, 2-bromo-2-methylpropane, 1,2-dichloro-1-fluoroethane, 1,1,1-trichloroethane, 2,2,2-trifluoroethanol, 1-chloro-3-fluoropropane, 1,1,1-trifluoro-2-propanol, 2,3-dichloro-1,1,1-trifluoropropane, perfluoromethylcyclohexane, ethylacetate, 1-butylamine, 1-chlorobutane, ethanol, butanone, 2,4-dimethylpentane, cyclohexane, n-propylformate, 2-ethoxy-butane, acetonitrile, pentafluoro-1-propanol, 2-propanol, tert-butanol, perfluoroheptane, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1,2-dichloroethane, 1-ethoxy-2-methylpropane, diisopropylamine, thiophene, 2-butanethiol, 1,1,1-trichloro-2,2,3,3-tetrafluoropropane, 1,2-dimethoxyethane, 3-methyl-2-butanamine, 1,1,3-trichloro-1,2,2,3-tetrafluoropropane, 1,3-dichloro-1,2,2-trifluoropropane, diethoxymethane, 1,1-dichloropropane, 2-methyl-1-propanethiol, 1,2-dichloro-2-fluoropropane, isopropylacetate, 2-iodopropane, di-n-propylether, 3-pentylamine, n-methylbutylamine, 2-bromobutane, 2,2-difluorotetrachloroethane, diethylsulfide, 1-ethoxybutane, 1,1,2,2-tetrachloro-1,2-difluoroethane, 1-fluorohexane, 1-methoxy-2-propanamine, 1,3-dichloro-1,2,3-trifluoropropane, 2,3-dichloro-1-propene, 2-methoxyethanamine, 2,2-difluoroethanol, 2-methylbutanal, 1,2-dichloropropane, propanol, tert-butylacetate, propionitrile, 3-chloropentane, trichloroacetaldehyde, 2-allyloxyethanol, butanethiol, isoamylchloride, 1-methoxy-pentane, ethylpropionate, 2-butanol, 1,2-dimethoxypropane, isopropyl-isobutylether, 1,1,1-trichloro-2,2,3-trifluoropropane, methylcyclohexane, 1-chloro-4-fluorobutane, 1-bromo-3-fluoropropane, 2,4,4-trimethyl-1-pentene, dioxane, 1-bromobutane, 3-pentanone, 1,1,2-trichloro-2-fluoroethane, 1,1-diethoxyethane, 2-pentanone, 2-methyl-2-butanol, 1-iodopropane, 2-methoxy-lpropanamine, 1,1,3-trichloro-1,2,2-trifluoropropane, 1,1,3-trichloro-2,2,3-trifluoropropane, trimethoxymethane, cis-1,3-dichloropropene, 2,2-dichlorobutane, n-pentylamine, 1,1-dichloro-2,2-difluoroethylmethylether, 2,2,4-trimethyl-2-pentene, perfluorooctane, 1,1,1,2-tetrachloro-2-fluoroethane, 1,3-dioxane, 3,3-dimethyl-2-butanone, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, 1-methylcyclohexene, toluene, trans-1,3-dichloropropene, sec-butylacetate, 2-fluorotoluene, 2,2-dimethyl-1-propanol, 1,1,1-trichloro-3-fluoropropane, n-methyl-1,2-ethanediamine, 2,2-diethoxypropane, 1,3,5-trioxane, 3,3,3-trichloro-1-propene, 1-chloro-3,3-dimethylbutane, pyridine, 2,3-dimethylhexane, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,1,3,3-tetrachloro-1,2,2-trifluoropropane, 1,2-propanediamine, 2,6-dimethyl-5-heptenal, perfluorobutanoicacid, 1,1,1,3-tetrachloro-2,2,3-trifluoropropane, 1-bromo-3-methylbutane, 1,3-dichloro-trans-2-butene, 1,3-dichloropropane, 1-(dimethylamino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2-propanol, 2-methoxyethanol, 1,2-dichlorobutane, 4,4,4-trifluorobutanol, 2-ethylbutylamine, perfluorononane, octane, diethylcarbonate, n-butylacetate, 1-pentanethiol, 1,2,2,3-tetrachloro-3,3-difluoropropane, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxyl-propanol, 1,2-dichloro-2-butene, 1-iodobutane, 1-ethoxy-2-propanol, hexanal, 4-methyl-2-pentanol, 1,2-dibromoethane, chlorobenzene, ethylcyclohexane, perfluorooctylbromide, bromoaceticacidmethylester, 1,1,2-trichloropropane, 1,2-octanediol, 4-methyl-2-hexanamine, hexylamine, 2-chloro-1-propanol, , 2-(dimethylamino)-ethanol, 1,3-dichlorobutane, cyclohexylamine, n-ethyl-2-dimethylaminoethylamine, ethoxyethanol, 3-hexanol, 2-hexanol, ethylbenzene, 2-methylpyrazine, 2-ethoxy-1-propanol, 1-pentanol, 1-methylpiperazine, n-ethyl-morpholine, 1,4-dimethylbenzene, 1,3-dimethylbenzene, 1,3-propanediamine, di-n-butylether, 3,3,4,4,5,5,6,6-octafluoro-1-pentanol,valeronitrile, (methyleneamino)acetonitrile, 1,2-dibromopropane, 2-heptanamine, 1,2,3-trichloropropene, 1,2,3-trimethylcyclohexane, 2,3-dimethylbutanol, 1-ethoxy-hexane, 1-chloro-3-bromopropane, perfluoro-n-decane, 3-furfural, n,n-diethylethylenediamine, 2,6-dimethylpyridine, 1,1,3,3-tetrachloro-1-fluoropropane, 4-methyl-2-hexanone, 1,2-dimethylbenzene, 1,1,2,2,3-pentchloro-3,3-difluoropropane, 1-methoxy-2-acetoxypropane, 1,1,1-triethoxyethane, styrene, 4-methylpyridine, n,n'-diethyl-1,2-ethanediamine, 2,6-dimethylmorpholine, 2-ethyl-1-butanol, 1,2-dichloropentane, 2-methyl-1-pentanol, methylhexanoate, 2-propoxyethanol, dimethylethanolamine, 1-propoxy-2-propanol. Avantageusement, ledit agent d'extraction organique peut avoir un facteur de séparation $S_{1,2}$ supérieur ou égal à 1,2 et/ou une capacité d'absorption $C_{2,S}$ supérieure ou égale à 0,40 ; et ledit agent d'extraction organique peut ainsi être chloroéthane, 3-chloro-1,1,1,2,2,3-hexafluoropropane, 1,1,1,2,2,3,3,4,4-nonafluorobutane, 2-chloro, 1,1,1,3,3,3-hexafluoropropane, 1,1,1,3,3-pentafluoropropane, ethylamine, bromofluoromethane, 1-bromo-1,2-difluoroethylene, 1-chloro-1,1,2,3,3,3-hexafluoropropane, 1-chloro-1,1,2,2-tetrafluoropropane, acetaldehyde, 1-chloro-1,1,2,2,3,3-hexafluoropropane, 1,1,1-trifluoro-2-propanone, 2-chloro-1,1,1,2-tetrafluoropropane, 1,1,1,2,3-pentafluoropropane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 2,2,2-trifluoroethylmethylether, furane, methylformate, isopropylamine, 1,1,2,2-tetrafluoroethylmethylether, 1,1,1,2,3,4,4,4, octafluorobutane, 2-methoxy-1-propene, heptafluoro-1-methoxypropane, diethylether, 1,2-epoxypropane, ethanethiol, 2-chloro-2-fluoropropane, ethoxy-ethene, 1-chloro-2,2-difluoroethane, ethylmethylamine, dimethylsulfide, 2-chloropropane, bromoethane, dimethoxymethane, 2-chloro-1,1,1,3,3-pentafluoropropane, 1-chloro-1,2,2-trifluoropropane, 2,2-dichloro-1,1,1,3,3-pentafluoropropane, 2-amino-2-methylpropane, methylcyclopropylether, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, n-propylamine, 1,1,2-trichloro-1,2,2-trifluoroethane, isopropylmethylamine, ethanedial, 1,2-dichloro-1,1,3,3,3-pentafluoropropane, 1-chloro-1,2,2,3-tetrafluoropropane, perfluorocyclohexane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 2-propanethiol, 1,2-dichloro-3,3,3-trifluoropropene, 1,1,1,2,2,3,4,5,5,5-decafluoropentane, 2-ethoxy-propane, 2,2-dichloro-1,1,1,3-tetrafluoropropane, 1-chloro-2,2-difluoropropane, methyl-t-butylether" diethylamine, propanone, perfluoro-n-hexane, methylacetate, ethyl1,1,2,2-tetrafluoroethylether, 4-methoxy-2-methyl-2-butanethiol, 2-bromopropane, 1,2-dichloro-1,2,3,3,4,4-hexafluorocyclobutane, 1,1-dichloro-2,2-difluoroethane, 2,2-dichloro-1,1,3,3-tetrafluoropropane, difluorodiethylsilane, 2-butanamine, 2,3-dichloro-1,1,1,2-tetrafluoropropane, n-methylpropylamine, 1,1-dichloro-1,2,2,3-tetrafluoropropane, tert-butylthiol, isobutanal, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, 2-chlorobutane, isopropylformate, diisopropylether, 1,3-dichloro-1,2,2,3-tetrafluoropropane, 1,2-dichloro-1,1,2,3-tetrafluoropropane, 2,3-dichloro-1,1,1,3-tetrafluoropropane, 1-bromopropane, 1,1-difluoro-1,2,2-trichloroethane, 1,1,2-trichloro-1,2-difluoroethane, methylglyoxal, 2-ethoxy-2-methyl-propane, 2-bromo-2-methylpropane, 1-chloro-3-fluoropropane, 1,1,1-trifluoro-2-propanol, 2,3-dichloro-1,1,1-trifluoropropane, perfluoromethylcyclohexane, ethylacetate, 1-butylamine, 1-chlorobutane, butanone, n-propylformate, 2-ethoxy-butane, pentafluoro-1-propanol, 2-propanol, tert-butanol, perfluoroheptane, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1-ethoxy-2-methylpropane, diisopropylamine, 2-butanethiol, 1,1,1-trichloro-2,2,3,3-tetrafluoropropane, 1,2-dimethoxyethane, 3-methyl-2-butanamine, 1,1,3-trichloro-1,2,2,3-tetrafluoropropane, 1,3-dichloro-1,2,2-trifluoropropane, diethoxymethane, 2-methyl-1-propanethiol, isopropylacetate, 2-iodopropane, di-n-propylether, 3-pentylamine, n-methylbutylamine, 2-bromobutane, diethylsulfide, 1-ethoxybutane, 1-fluorohexane,, 1-methoxy-2-propanamine, 1,3-dichloro-1,2,3-trifluoropropane, 2-methoxyethanamine, 2, methylbutanal, propanol, tert-butylacetate, propionitrile, 3-chloropentane, 2-allyloxyethanol, butanethiol, isoamylchloride, 1-methoxy-pentane, ethylpropionate, 2-butanol, 1,2-dimethoxypropane, isopropyl-isobutyl-ether, 1,1,1-trichloro-2,2,3-trifluoropropane, 1-chloro-4-fluorobutane, 1-bromo-3-fluoropropane, 2,4,4-trimethyl-1-pentene, dioxane, 1-bromobutane, 3-pentanone, 1,1-diethoxyethane, 2-pentanone, 2-methyl-2-butanol, 2-methoxy-lpropanamine, 1,1,3-trichloro-1,2,2-trifluoropropane, 1,1,3-trichloro-2,2,3-trifluoropropane, trimethoxymethane, n-pentylamine, 1,1-dichloro-2,2-difluoroethylmethylether, 2,2,4-trimethyl-2-pentene, perfluorooctane, 1,3-dioxane, 3,3-dimethyl-2-butanone, piperidine, isobutanol, 2-bromo-2-methylbutane, dipropylamine, 2,2,3,3-tetraflouro-1-propanol, 2-ethoxyethanamine, triethylfluorosilane, 1-methylcyclohexene, toluene, sec-butylacetate, 2-fluorotoluene, 2,2-dimethyl-1-propanol, n-methyl, 1,2-ethanediamine, 2,2-diethoxypropane, 1,3,5-trioxane, 1-chloro-3,3-dimethylbutane, pyridine, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, sec-butyl-tert-butylether, 1-methoxy2-propanol, 1,1,3,3-tetrachloro-1,2,2-trifluoropropane, 1,2-propanediamine, 2,6-dimethyl-5-heptenal, perfluorobutanoicacid, 1,1,1,3-tetrachloro-2,2,3-trifluoropropane, 1-bromo-3-methylbutane, 1-(dimethylamino)-2-propanol, tetrahydrothiophene, 3-methyl-3-pentanol, 1,1-diethoxypropane, 2-methoxyethanol, 4,4,4-trifluorobutanol, 2-ethylbutylamine, perfluorononane, diethylcarbonate, n-butylacetate, 1-pentanethiol, 2-chloro-1,1-dimethoxyethane, 2-hexanone, n-ethylethylenediamine, 5-hexen-2-one, 1,1-diethoxy-n,n-dimethylmethanamine, 2-methylpyridine, 1-bromopentane, 2-methoxyl-propanol, 1-ethoxy-2-propanol, hexanal, 4-methyl-2-pentanol, perfluorooctylbromide, 1,2-octanediol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 3-hexanol, 2-hexanol, ethylbenzene, 2-methylpyrazine, 2-ethoxy-1-propanol, 1-pentanol, 1-methylpiperazine, n-ethyl-morpholine, 1,4-dimethylbenzene, 1,3-dimethylbenzene, 1,3-propanediamine, di-n-butylether, 3,3,4,4,5,5,6,6-octafluoro-1-pentanol, valeronitrile, (methyleneamino)acetonitrile, 2-heptanamine, 2,3, dimethylbutanol, 1-ethoxy-hexane, perfluoro-n-decane, 3-furfural, n,n-diethylethylenediamine, 2,6-dimethylpyridine, 4-methyl-2-hexanone, 1,2-dimethylbenzene, 1-methoxy-2-acetoxypropane, 1,1,1-triethoxyethane, styrene, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, dimethylethanolamine, 1-propoxy-2-propanol.

De préférence, ledit agent d'extraction organique peut avoir un facteur de séparation $S_{1,2}$ supérieur ou égal à 1,4 et/ou une capacité d'absorption $C_{2,S}$ supérieure ou égale à 0,6 ; et ledit agent d'extraction organique peut ainsi être Ethylamine, acetaldehyde, 1,1,1,3,3-pentafluoropropane, 1,1,1-trifluoro-2-propanone, 1,1,1,2,3-pentafluoropropane, 2,2,2-trifluoroethylmethylether, methylformate, isopropylamine, 1,1,2,2-tetrafluoroethylmethylether, 2-methoxy-1-propene, diethylether, 1,2-epoxypropane, ethoxy-ethene, ethylmethylamine, dimethoxymethane, 1-chloro-1,2,2-trifluoropropane, 2-amino-2-methylpropane, methylcyclopropylether, 3-chloro-1,1,1-trifluoropropane, n-propylamine, isopropylmethylamine, ethanedial, 1-chloro-1,2,2,3-tetrafluoropropane, 3-chloro-1,1,1,3-tetrafluoropropane, 2-chloro-1,1,1,3-tetrafluoropropane, 2-ethoxy-propane, methyl-t-butylether, diethylamine, propanone, methylacetate, ethyl1,1,2,2-tetrafluoroethylether, 4-methoxy-2-methyl-2-butanethiol, difluorodiethylsilane, 2-butanamine, n-methylpropylamine, isobutanal, tetrahydrofurane, 1,3-dichloro-1,1,2,2-tetrafluoropropane, isopropylformate, diisopropylether, methylglyoxal, 2-ethoxy-2-methyl-propane, 2,3-dichloro-1,1,1-trifluoropropane, ethylacetate, 1-butylamine, butanone, n-propylformate, 2-ethoxy-butane, 1,3-dichloro-1,1,2-trifluoropropane, 1-methoxy-2-methyl-butane, 1,1-dichloro-2,2,3-trifluoropropane, 2,2-dimethoxypropane, 1-ethoxy-2-methylpropane, diisopropylamine, 1,2-dimethoxyethane, 3-methyl-2-butanamine, 1,3-dichloro-1,2,2-trifluoropropane, diethoxymethane, isopropylacetate, di-n-propylether, 3-pentylamine, n-methylbutylamine, diethylsulfide, 1-ethoxybutane, 1-fluorohexane, 1-methoxy-2-propanamine, 1,3-dichloro-1,2,3-trifluoropropane, 2-methoxyethanamine, 2-methylbutanal, tert-butylacetate, propionitrile, 2-allyloxyethanol, 1-methoxy-pentane, ethylpropionate, 1,2-dimethoxypropane, isopropyl-isobutyl-ether, dioxane, 3-pentanone, 1,1-diethoxyethane, 2-pentanone, 2-methoxy-1propanamine, trimethoxymethane, n-pentylamine, 1,3-dioxane, 3,3-dimethyl-2-butanone, piperidine, dipropylamine, 2-ethoxyethanamine, triethylfluorosilane, sec-butylacetate, n-methyl-1,2-ethanediamine, 2,2-diethoxypropane, pyridine, n-methylmorpholine, 4-methyl-2-pentanone, 1,2-diaminoethane, isobutyl-tert-butylether, butyronitrile, sec-butyl-tert-butylether, 1-methoxy2-propanol, 1,2-propanediamine, 2,6-dimethyl-5-heptenal, perfluorobutanoicacid, 1-(dimethylamino)-2-propanol, 3-methyl-3-pentanol, 1,1-diethoxypropane, 2-ethylbutylamine, diethylcarbonate, n-butylacetate, 2-hexanone, n-ethylethylenediamine, 5-hexen-2-one, 1,1-diethoxy-n,n-dimethylmethanamine, 2-methylpyridine, 2-methoxyl-propanol, 1-ethoxy-2-propanol, hexanal, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-methylpyrazine, 2-ethoxy-1-propanol, 1-methylpiperazine, n-ethyl-morpholine, 1,3-propanediamine, di-n-butylether, 3,3,4,4,5,5,6,6-octafluoro-1-pentanol, valeronitrile, 2-heptanamine, 1-ethoxy-hexane, n,n-diethylethylenediamine, 2,6-dimethylpyridine, 4-methyl-2-hexanone, 1-methoxy-2-acetoxypropane, 1,1,1-triethoxyethane, 4-methylpyridine, n,n'-diethyl-1,2-ethanediamine, 2,6-dimethylmorpholine, methylhexanoate, 2-propoxyethanol, 1-propoxy-2-propanol.

En particulier, ledit agent d'extraction organique peut avoir un facteur de séparation $5_{1,2}$ supérieur ou égal à 1,6 et/ou une capacité d'absorption $C_{2,S}$ supérieure ou égale à 0,80 ; et ledit agent d'extraction organique peut ainsi être éthylamine, 1,1,1-trifluoro-2-propanone, methylformate, isopropylamine, 2-methoxy-1-propene, diethylether, 1,2-epoxypropane, ethoxy-ethene, ethylmethylamine, dimethoxymethane, 2-amino-2-methylpropane, methylcyclopropylether, n-propylamine, isopropylmethylamine, 2-ethoxy-propane, methyl-t-butylether, diethylamine, propanone, methylacetate, 2-butanamine, n-methylpropylamine, isobutanal, tetrahydrofurane, isopropylformate, diisopropylether, 2-ethoxy-2-methyl-propane, ethylacetate, 1-butylamine, butanone, n-propylformate, 2-ethoxy-butane, 1-methoxy-2-methyl-butane, 2,2-dimethoxypropane, 1-ethoxy-2-methylpropane, 1,2-dimethoxyethane, 3-methyl-2-butanamine, diethoxymethane, isopropylacetate, di-n-propylether, 3-pentylamine, n, methylbutylamine, 1-ethoxybutane, 1-methoxy-2-propanamine, 2-methoxyethanamine, 2-methylbutanal, tert-butylacetate, 1-methoxy-pentane, ethylpropionate, 1,2-dimethoxypropane, dioxane, 3-pentanone, 1,1-diethoxyethane, 2-pentanone, 2-methoxy-1propanamine, trimethoxymethane, n-pentylamine, 1,3-dioxane, 3,3-dimethyl-2-butanone, piperidine, 2-ethoxyethanamine, sec-butylacetate, n-methyl-1,2-ethanediamine, 2,2-diethoxypropane, 4-methyl-2-pentanone, 1,2-diaminoethane, butyronitrile, 1-methoxy2-propanol, 1,2-propanediamine, 2,6-dimethyl-5-heptenal, 1-(dimethylamino)-2-propanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxyl-propanol, 1-ethoxy-2-propanol, hexanal, 4-methyl-2-hexanamine, hexylamine, methoxycyclohexane, 2-(dimethylamino)-ethanol, cyclohexylamine, n-ethyl-2-dimethylaminoethylamine, 2-methylpyrazine, 2-ethoxy-1-propanol, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1-methoxy-2-acetoxypropane, 1,1,1-triethoxyethane, 4-methylpyridine, n,n'-diethyl-1,2-ethanediamine, 2,6-dimethylmorpholine, methylhexanoate, 2-propoxyethanol, 1-propoxy-2-propanol. Ledit agent d'extraction organique peut avoir un facteur de séparation $S_{1,2}$ supérieur ou égal à 1,6 et/ou une capacité d'absorption $C_{2,S}$ supérieure ou égale à 0,80 ; et être sélectionné parmi éthylamine, isopropylamine, diethylether, ethoxy-ethene, dimethoxymethane, n-propylamine, methyl-t-butylether, diethylamine, propanone, methylacetate, isobutanal, tetrahydrofurane, isopropylformate, diisopropylether, 2-ethoxy-2-methyl-propane, ethylacetate, butanone, diethoxymethane, isopropylacetate, 3-pentylamine, 2-methoxyethanamine, tert-butylacetate, dioxane, 3-pentanone, 1,1-diethoxyethane, 2-pentanone, trimethoxymethane, n-pentylamine, 1,3-dioxane, 3,3-dimethyl-2-butanone, sec-butylacetate, 4-methyl-2-pentanone, 1,2-diaminoethane, 1-methoxy2-propanol, diethylcarbonate, n-butylacetate, 1-ethoxy-2-propanol, hexanal. Avantageusement, ledit agent d'extraction organique peut avoir un facteur de séparation $S_{1,2}$ supérieur ou égal à 1,6 et/ou une capacité d'absorption $C_{2,S}$ supérieure ou égale à 0,80 ; et être sélectionné parmi Ethylamine, isopropylamine, diethylether, dimethoxymethane, n-propylamine, diethylamine, ,diisopropylether, 2-ethoxy-2-methyl-propane, butanone, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal. De préférence, ledit agent d'extraction organique peut avoir un facteur de séparation $S_{1,2}$ supérieur ou égal à 1,6 et/ou une capacité d'absorption $C_{2,S}$ supérieure ou égale à 0,80 ; et être sélectionné parmi Ethylamine, isopropylamine, diethylether, dimethoxymethane, n-propylamine, diethylamine, diisopropylether, 2-ethoxy-2-methyl-propane, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal.

Selon un mode de réalisation préféré, ladite troisième composition comprenant ledit agent d'extraction organique et ledit au moins un des composés sélectionné parmi le groupe consistant en 1,1-difluoroéthane (152a), chloropentafluoroéthane (115), trans-1,3,3,3-tetrafluoro-1-propene (1234ze-E) ; de préférence la troisième composition comprenant ledit agent d'extraction organique et trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115) ; peut être soumise à une distillation pour séparer d'une part l'agent d'extraction organique et d'autre part ledit au moins un des composés sélectionné parmi le groupe consistant en 1,1-difluoroéthane (152a), chloropentafluoroéthane (115), trans-1,3,3,3-tetrafluoro-1-propene (1234ze-E) ; de préférence pour séparer d'une part l'agent d'extraction organique et d'autre part trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115).

Selon un mode de réalisation particulier, le courant comprenant le 2,3,3,3-tetrafluoropropène séparé à l'étape b) du présent procédé est purifié ultérieurement ou liquéfié et stocké. Dans ce mode de réalisation, des traces d'agent d'extraction organique peuvent être présentes dans le courant comprenant le 2,3,3,3-tetrafluoropropène. Les traces d'agent d'extraction organique dans ledit courant sont inférieures à 10 ppm, de préférence inférieures à 1 ppm, en particulier inférieures à 500 ppb, plus particulièrement inférieures à 100 ppb.

Le présent procédé permet donc de purifier le 2,3,3,3-tetrafluoro-1-propène. Avantageusement, la teneur en au moins un des composés sélectionné parmi le groupe consistant en 1,1-difluoroéthane (152a), chloropentafluoroéthane (115), trans-1,3,3,3-tetrafluoro-1-propene (1234ze-E) dans le courant comprenant du 2,3,3,3-tetrafluoro-1-propène, obtenu à l'étape b) du présent procédé de purification est inférieure à la teneur de celui-ci ou de ceux-ci dans ladite première composition. De préférence, la teneur en trans-1,3,3,3-tetrafluoro-1-propene (1234ze-E) dans le courant comprenant du 2,3,3,3-tetrafluoro-1-propène, obtenu à l'étape b) du présent procédé de purification est inférieure à la teneur trans-1,3,3,3-tetrafluoro-1-propene (1234ze-E) dans ladite première composition.

Par exemple, la teneur en l'un quelconque des composés peut être diminuée de 50%, avantageusement de 75%, de préférence de 90%, en particulier de 95%, plus particulièrement de 98%. Avantageusement, la teneur en au moins deux ou en l'ensemble desdits composés sélectionné parmi le groupe consistant en 1,1-difluoroéthane (152a), chloropentafluoroéthane (115), trans-1,3,3,3-tetrafluoro-1-propene (1234ze-E) peut être diminuée de 50%, avantageusement de 75%, de préférence de 90%, en particulier de 95%, plus particulièrement de 98%. De préférence, la teneur en trans-1,3,3,3-tetrafluoro-1-propene (1234ze-E) peut être diminuée de 50%, avantageusement de 75%, de préférence de 90%, en particulier de 95%, plus particulièrement de 98%. Les teneurs sont exprimées en pourcentage en poids.

De préférence, le courant comprenant le 2,3,3,3-tetrafluoro-1-propène obtenu à l'étape b) du présent procédé de purification peut être dépourvu d'au moins un, d'au moins deux ou de l'ensemble des composés sélectionné parmi le groupe consistant en 1,1-difluoroéthane (152a), chloropentafluoroéthane (115), trans-1,3,3,3-tetrafluoro-1-propene (1234ze-E) lorsque celui-ci ou ceux-ci sont présents dans ladite première composition. En particulier, le courant comprenant le 2,3,3,3-tetrafluoro-1-propène obtenu à l'étape b) du présent procédé de purification peut être dépourvu de trans-1,3,3,3-tetrafluoro-1-propene (1234ze-E). Le terme « dépourvu » signifie que le courant comprenant le 2,3,3,3-tetrafluoro-1-propène (1234yf) comprend moins de 50 ppm, avantageusement moins de 20 ppm, de préférence moins de 10 ppm du composé considéré sur base du poids total du courant.

Selon un mode de réalisation préféré, ladite première composition mise en oeuvre à l'étape a) du présent procédé peut être préalablement purifiée avant d'être utilisée. En effet, si ladite première composition comprend des impuretés ayant un point d'ébullition inférieur au point d'ébullition du 2,3,3,3-tetrafluoro-1-propène et optionnellement des impuretés lourdes, le présent procédé peut comprendre préalablement à l'étape a) les étapes :

i') mise en oeuvre ou fourniture d'une composition comprenant 2,3,3,3-tetrafluoro-1-propène, des impuretés ayant un point d'ébullition inférieur au point d'ébullition du 2,3,3,3-tetrafluoro-1-propène, et au moins un des composés sélectionné parmi le groupe consistant en 1,1-difluoroéthane (152a), chloropentafluoroéthane (115), trans-1,3,3,3-tetrafluoro-1-propene (1234ze-E), de préférence trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115), et optionnellement des impuretés lourdes ;

ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E), de préférence un premier courant comprenant 2,3,3,3-tetrafluoro-1-propène, trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115), et optionnellement des impuretés lourdes, récupéré en bas de colonne de distillation ;

iii') optionnellement, distillation dudit premier courant récupéré en bas de colonne de distillation à l'étape ii') pour récupérer en tête de colonne un second courant comprenant du 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E) ; de préférence un second courant comprenant du 2,3,3,3-tetrafluoro-1-propène, trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115) ; et en bas de colonne de distillation un courant comprenant les impuretés lourdes ;

ledit premier courant récupéré à l'étape ii') ou ledit second courant récupéré à l'étape iii') corresponde à ladite première composition mise en oeuvre à l'étape a).

Les impuretés lourdes sont telles que définis ci-dessus dans la présente demande.

Selon un second aspect, la présente invention fournit un procédé de production du 2,3,3,3-tetrafluoro-1-propène. En outre, ce procédé peut inclure sa purification. Ainsi, la présente invention fournit un procédé de production de 2,3,3,3-tetrafluoro-1-propène comprenant les étapes de :

A) fluoration en présence d'un catalyseur d'un composé de formule (I) $CX(Y)_2$-$CX(Y)_m$-$CH_mXY$ dans laquelle X et Y représentent indépendamment un atome d'hydrogène, de fluor ou de chlore et m = 0 ou 1 ; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E), de préférence un courant comprenant du 2,3,3,3-tetrafluoro-1-propène, trans-1,3,3,3-tetrafluoro-1-propene (1234ze-E) et optionnellement ou non un composés choisis parmi le groupe consistant en 1,1-difluoroéthane (152a) et chloropentafluoroéthane (115) ;

C) mise en oeuvre du procédé de purification du 2,3,3,3-tetrafluoro-1-propène selon la présente invention à partir du courant récupéré à l'étape B).

Selon un mode de réalisation préféré, le procédé de production de 2,3,3,3-tetrafluoro-1-propène comprenant les étapes de :

A) fluoration en présence d'un catalyseur d'un composé de formule (I) $CX(Y)_2$-$CX(Y)_m$-$CH_mXY$ dans laquelle X et Y représentent indépendamment un atome d'hydrogène, de fluor ou de chlore et m = 0 ou 1 et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E), de préférence un courant comprenant 2,3,3,3-tetrafluoro-1-propène, trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115) ;

C) mise en oeuvre du procédé de purification du 2,3,3,3-tetrafluoro-1-propène à partir du courant récupéré à l'étape B) et comprenant les étapes de :

a) mise en contact de ladite première composition avec au moins un agent d'extraction organique pour former une seconde composition ;

b) distillation extractive de ladite seconde composition pour former :

i) une troisième composition comprenant ledit agent d'extraction organique et ledit au moins un des composés sélectionné parmi le groupe consistant en 1,1-difluoroéthane (152a), chloropentafluoroéthane (115), trans-1,3,3,3-tetrafluoro-1-propene (1234ze-E), de préférence une troisième composition comprenant ledit agent d'extraction organique, trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115); et

ii) un courant comprenant le 2,3,3,3-tétrafluoro-1-propène,

c) récupération et séparation de ladite troisième composition pour former un courant comprenant ledit agent d'extraction organique et un courant comprenant ledit au moins un des composés sélectionné parmi le groupe consistant en 1,1-difluoroéthane (152a), chloropentafluoroéthane (115), trans-1,3,3,3-tetrafluoro-1-propene (1234ze-E), de préférence un courant comprenant ledit agent d'extraction organique et un courant comprenant trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115).

Selon un mode de réalisation particulier, le courant récupéré à l'étape B) du procédé de production du 2,3,3,3-tétrafluoro-1-propène peut comprendre de l'acide fluorhydrique. Dans ce cas, préalablement à l'étape C), le courant récupéré à l'étape B) peut subir une distillation préalable B1') pour éliminer HF en bas de colonne de distillation. Un courant comprenant 2,3,3,3-tétrafluoro-1-propène, et au moins un des composés choisi parmi le groupe consistant en 1,1-difluoroéthane (152a), chloropentafluoroéthane (115), trans-1,3,3,3-tetrafluoro-1-propene (1234ze-E), de préférence un courant comprenant 2,3,3,3-tétrafluoro-1-propène, trans-1,3,3,3-tetrafluoro-1-propene (1234ze-E) et optionnellement ou non au moins un des composés choisi parmi le groupe consistant en 1,1-difluoroéthane (152a) et chloropentafluoroéthane (115), est récupéré en tête de colonne de distillation. Ce dernier courant récupéré en tête de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut comprendre des impuretés ayant un point d'ébullition inférieur au point d'ébullition du 2,3,3,3-tetrafluoro-1-propène. Dans ce cas, préalablement à l'étape C), le courant récupéré à l'étape B) peut subir une distillation préalable B2'), subséquente ou non à l'étape B1'), pour éliminer en tête de colonne de distillation lesdites impuretés ayant un point d'ébullition inférieur au point d'ébullition du 2,3,3,3-tetrafluoro-1-propène et former un premier courant comprenant 2,3,3,3-tetrafluoro-1-propène, et au moins un des composés choisi parmi le groupe consistant en 1,1-difluoroéthane (152a), chloropentafluoroéthane (115), trans-1,3,3,3-tetrafluoro-1-propene (1234ze-E) , de préférence un premier courant comprenant 2,3,3,3-tetrafluoro-1-propène, trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115), récupéré en bas de colonne de distillation. Ce dernier courant récupéré en bas de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut comprendre des impuretés lourdes. Dans ce cas, préalablement à l'étape C), le courant récupéré à l'étape B) peut subir une distillation préalable B3'), subséquente ou non à l'étape B1') et/ou B2'), pour éliminer en bas de colonne de distillation lesdites impuretés lourdes ; et former un premier courant comprenant 2,3,3,3-tetrafluoro-1-propène, et au moins un des composés choisi parmi le groupe consistant en 1,1-difluoroéthane (152a), chloropentafluoroéthane (115), trans-1,3,3,3-tetrafluoro-1-propene (1234ze-E), de préférence un premier courant comprenant 2,3,3,3-tetrafluoro-1-propène, trans-1,3,3,3-tetrafluoro-1-propene (1234ze-E) et optionnellement ou non au moins un des composés choisi parmi le groupe consistant en 1,1-difluoroéthane (152a), chloropentafluoroéthane (115), récupéré en tête de colonne de distillation. Ce dernier courant récupéré en tête de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut également comprendre HCl. L'acide chlorhydrique peut être récupéré par distillation avant ou après l'étape B1' indépendamment des autres étapes du procédé.

Plus particulièrement, l'étape A) est effectuée à partir de 1,1,2,3-tetrachloropropène, le 2,3,3,3,-tetrachloropropène, le 1,1,3,3-tetrachloropropène, le 1,3,3,3-tetrachloropropène, le 1,1,1,2,3-pentachloropropane, le 1,1,1,3,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoropropane, de préférence à partir de 1,1,1,2,3-pentachloropropane, 1,1,2,3,tetrachloropropène, du 1,1,1,2,2-pentafluoropropane et/ou du 2-chloro-3,3,3-trifluoro-1-propène ; en particulier à partir du 1,1,1,2,3-pentachloropropane (240db).

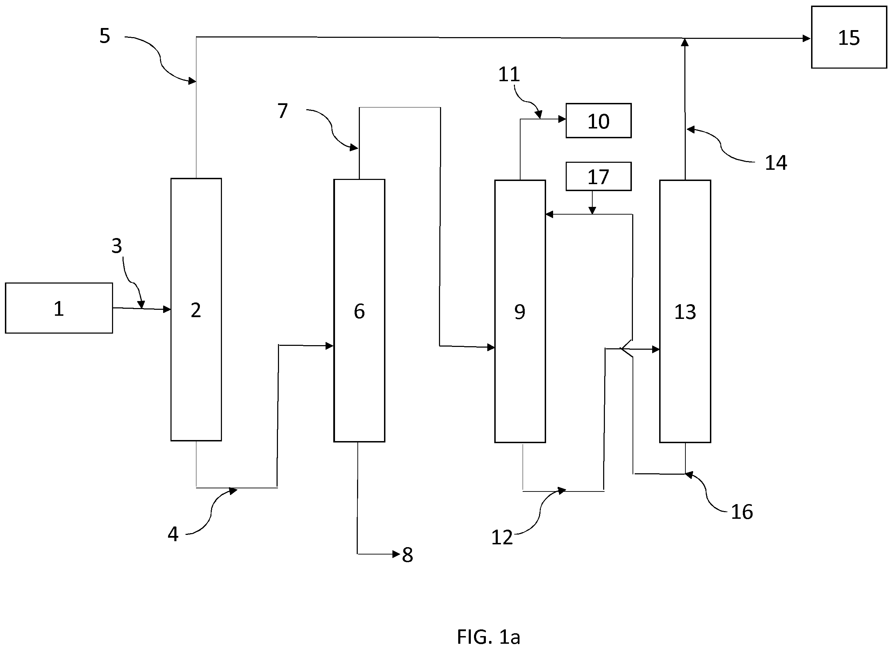

La figure 1a représente un schéma simplifié d'un dispositif mettant en oeuvre un procédé de purification du 2,3,3,3-tetrafluoro-1-propene selon un mode de réalisation particulier de l'invention. Le mélange issu de la réaction de fluoration, en présence d'un catalyseur, d'un composé de formule $(CX_nY_{3-n})CHXCH_{m+1}X_{2-m}$ (I) et/ou de fluoration catalytique, en présence d'un catalyseur, d'un composé de formule $(CX_nY_{3-n})CH_pX_{1-p}=CH_mX_{2-m}$ (II) est obtenu en 1. Le mélange comprend, dans ce mode de réalisation particulier, 2,3,3,3-tetrafluoro-1-propene, trans-1,3,3,3-tetrafluoro-1-propene (1234ze-E) et au moins un des composés choisi parmi le groupe consistant en 1,1-difluoroéthane (152a) et chloropentafluoroéthane (115), des impuretés ayant un point d'ébullition inférieur au point d'ébullition du 2,3,3,3-tetrafluoro-1-propene et des impuretés lourdes. Le mélange est transféré dans une colonne de distillation 2 par le conduit 3. Les impuretés ayant un point d'ébullition inférieur au point d'ébullition du 2,3,3,3-tetrafluoro-1-propene sont récupérées en tête de la colonne de distillation 2 et acheminées par le conduit 5 à un incinérateur ou un dispositif de purification 15. Le résidu obtenu en bas de colonne de distillation et comprenant les autres constituants du mélange est acheminé vers une seconde colonne de distillation 6 par le conduit 4. La distillation effectuée en 6 vise à séparer les impuretés lourdes des autres constituants du mélange. Les conditions opératoires de distillation sont donc adaptées à ce but. Les impuretés lourdes sont récupérées en bas de colonne de distillation 8 et les autres constituants sont récupérés en tête de colonne de distillation et acheminés vers la colonne de distillation extractive 9 via le conduit 7. La distillation extractive effectuée en 9 vise à séparer le 2,3,3,3-tetrafluoro-1-propene des autres constituants du mélange mentionnés ci-dessus. Le dispositif de distillation extractive 9 est alimenté par l'agent d'extraction organique 17 sélectionné selon la méthode décrite dans la présente demande. Le 2,3,3,3-tetrafluoro-1-propene est récupéré en tête du dispositif de distillation extractive 9 pour être stocké ou purifié en 10 via le conduit 11. Le mélange récupéré en bas du dispositif de distillation extractive 9 comprend notamment l'agent d'extraction organique 17, trans-1,3,3,3-tetrafluoro-1-propene (1234ze-E) et au moins un des composés suivants 1,1-difluoroéthane (152a) ou chloropentafluoroéthane (115). Le mélange récupéré en bas du dispositif de distillation extractive 9 est acheminé par le conduit 12 vers un dispositif de distillation 13 visant à séparer l'agent d'extraction organique des autres composés présents. L'agent d'extraction organique est récupéré en bas du dispositif de distillation et recyclé par le conduit 16 vers le dispositif de distillation extractive 9. Les composés récupérés en tête du dispositif de distillation 13 sont acheminés par le conduit 14 vers le conduit 5 pour être incinérés ou purifiés en 15.

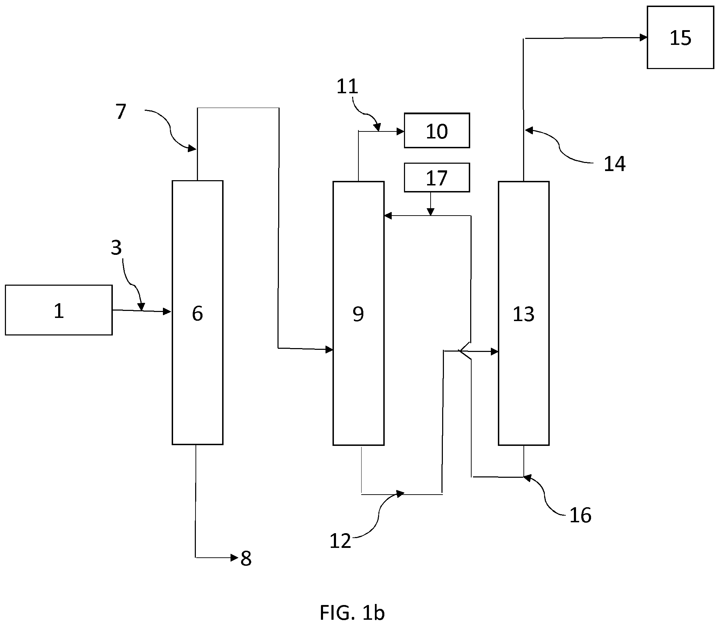

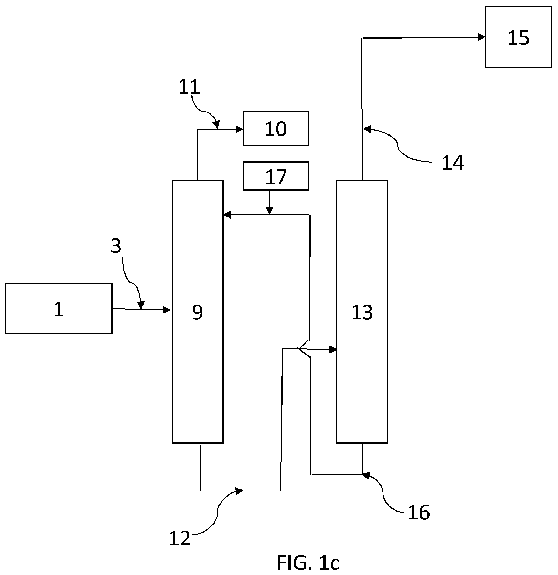

Le mélange fourni en 1 peut être dépourvu d'impuretés ayant un point d'ébullition inférieur à celui du 2,3,3,3-tetrafluoro-1-propène. Dans ce cas, comme illustré à la Fig. 1b, le mélange 1 est acheminé par le conduit 3 vers la colonne de distillation 6 pour être traité comme expliqué ci-dessus en relation avec la Fig. 1a. Dans un autre mode de réalisation particulier illustré à la Fig. 1c, le mélange 1 peut comprendre le 2,3,3,3-tetrafluoro-1-propene, trans-1,3,3,3-tetrafluoro-1-propene (1234ze-E), et au moins un des composés suivants 1,1-difluoroéthane (152a) ou chloropentafluoroéthane (115). Dans ce cas, le mélange est acheminé directement vers la colonne de distillation extractive 9 pour y être traité comme expliqué ci-dessus en relation avec la Fig. 1a.

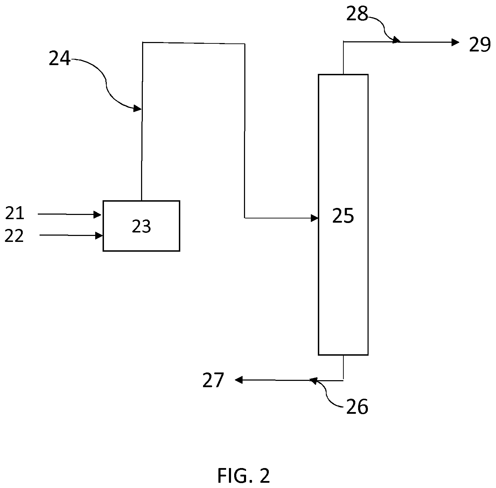

La Fig. 2 illustre schématiquement un dispositif mettant en oeuvre un procédé de production du 2,3,3,3-tetrafluoropropène selon un mode de réalisation particulier de la présente invention. L'acide fluorhydrique 21 est mis en contact avec du 1,1,1,2,3-pentachloropropane (240db) 22 dans un réacteur 23. Le mélange obtenu et comprenant 2,3,3,3-tetrafluoro-1-propène, trans-1,3,3,3-tetrafluoro-1-propene (1234ze-E) et au moins un des composés suivants 1,1-difluoroéthane (152a) ou chloropentafluoroéthane (115), est récupéré en sortie de réacteur et acheminé vers une colonne de distillation 25 par le conduit 24. Le mélange peut aussi comprendre HCl, HF et des impuretés lourdes ou ayant un point d'ébullition inférieur à celui du 2,3,3,3-tetrafluoro-1-propène. Le courant obtenu en bas de colonne de distillation comprenant HF et éventuellement des impuretés lourdes est acheminé vers le dispositif de purification 27 via le conduit 26 pour purifier HF qui sera recyclé éventuellement en 23. Les autres constituants du mélange sont acheminés via le conduit 28 vers un dispositif de purification 29 de purification du 2,3,3,3-tetrafluoro-1-propène. Le dispositif de purification 29 peut être l'un quelconque des dispositifs illustrés aux Fig. 1a-1b.

Le catalyseur utilisé dans le présent procédé de production de 2,3,3,3-tétrafluoropropène peut être par exemple à base d'un métal comprenant un oxyde de métal de transition ou un dérivé ou un halogénure ou un oxyhalogénure d'un tel métal. On peut citer par exemple $FeCl_3$, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple $AlF_3$ et $Al_2O_3$, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 I.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :

- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bar, de préférence de 2 à 18 bar et plus particulièrement de 3 à 15 bars;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

**Méthode de sélection de l'agent d'extraction organique**

La sélection de l'agent d'extraction organique est déterminée par l'utilisation du modèle Cosmo-RS implémenté dans le logiciel COSMOTHERM. Pour ce couple binaire sélectionné, un facteur de séparation est calculé pour chacun des solvants étudiés par l'équation suivante :

$$S_{1,2} = (\gamma_{1,S} * P1)/(\gamma_{2,S} * P2)$$

dans laquelle

$\gamma_{1,S}$ représente le coefficient d'activité du premier composé 1 dans l'agent d'extraction organique considéré à dilution infinie,

P1 représente la pression de vapeur saturante du premier composé 1,

$\gamma_{2,S}$ représente le coefficient d'activité du second composé 2 du couple binaire dans l'agent d'extraction organique considéré à dilution infinie,

P2 représente la pression de vapeur saturante du second composé.

Une capacité d'absorption est également calculée pour chacun des solvants étudiés et pour un couple binaire (1,2) considéré. La capacité d'absorption est calculée par la formule $C_{2,S} = 1/(\gamma_{2,S})$ dans laquelle $y_{2,s}$ représente le coefficient d'activité du second composé du couple binaire considéré dans ledit agent d'extraction organique étudié à dilution infinie.

Les calculs sont répétés pour chaque agent d'extraction organique étudié. Des valeurs minimales de facteur de séparation et de capacité d'absorption sont identifiées afin de permettre une séparation suffisante entre le premier composé et le second composé du couple binaire (1,2) considéré. La pression de vapeur saturante est considérée pour une température de 25°C.

**Exemple**

Pour purifier le 2,3,3,3-tetrafluoro-1-propene, le couple binaire 2,3,3,3-tetrafluoro-1-propene / trans-1,3,3,3-tetrafluoro-1-propene (1234ze-E) est considéré pour sélectionner l'agent d'extraction organique (Voir tableau 1 ci-dessous). Le mélange à purifier comprend 2,3,3,3-tetrafluoro-1-propene, 1,1-difluoroéthane (152a), chloropentafluoroéthane (115), et trans-1,3,3,3-tetrafluoro-1-propene (1234ze-E). Les distillations extractives sont effectuées avec les agents d'extraction organique ayant un facteur de séparation supérieur ou égal à 1,6 et une capacité d'absorption supérieure ou égale à 0,80. Le 2,3,3,3-tetrafluoro-1-propene a été extrait et la teneur dans les autres constituants sont diminuées, principalement en trans-1,3,3,3-tetrafluoro-1-propene (1234ze-E).

Tableau 1 - Capacité et Facteur de séparation de l'agent d'extraction organique

| Agent extraction organique | Capacité d'absorption | Facteur de séparation |
|---|---|---|
| Ethylamine | 1,95 | 2,92 |
| Isopropylamine | 1,85 | 2,56 |
| Diethylether | 1,48 | 1,79 |
| Dimethoxyméthane | 1,37 | 1,95 |
| n-propylamine | 1,86 | 2,61 |
| diéthylamine | 1,57 | 1,88 |
| Diisopropylether | 1,34 | 1,60 |
| 2-ethoxy-2-méthylpropane | 1,27 | 1,62 |
| Diethoxyméthane | 1,41 | 1,86 |
| Isopropylacétate | 1,42 | 2,15 |
| 3-pentylamine | 1,63 | 2,05 |
| 2-methoxyethanamine | 1,98 | 3,19 |
| tert-butylacetate | 1,37 | 2,10 |
| dioxane | 1,09 | 2,24 |
| Trimethoxyméthane | 1,28 | 2,11 |
| n-pentylamine | 1,64 | 2,31 |
| 1,3-dioxane | 1,09 | 2,23 |
| sec-butylacétate | 1,45 | 2,12 |
| 1,2-diaminoéthane | 1,29 | 4,43 |
| 1-methoxy-2-propanol | 0,86 | 2,31 |

(suite)

| Agent extraction organique | Capacité d'absorption | Facteur de séparation |
|---|---|---|
| n-butylacétate | 1,35 | 2,14 |
| 1-éthoxy-2-propanol | 1,12 | 2,27 |
| hexanal | 1,09 | 1,98 |

**Revendications**

1. Procédé de purification du 2,3,3,3-tétrafluoro-1-propène (1234yf) à partir d'une première composition comprenant du 2,3,3,3-tétrafluoro-1-propène et au moins un des composés sélectionné parmi le groupe consistant en 1,1-difluoroéthane (152a), chloropentafluoroéthane (115), trans-1,3,3,3-tetrafluoro-1-propene (1234ze-E), ledit procédé comprenant les étapes de:

   a) mise en contact de ladite première composition avec au moins un agent d'extraction organique pour former une seconde composition ;
   b) distillation extractive de ladite seconde composition pour former :

   i) une troisième composition comprenant ledit agent d'extraction organique et ledit au moins un des composés sélectionné parmi le groupe consistant en 1,1-difluoroéthane (152a), chloropentafluoroéthane (115), trans-1,3,3,3-tetrafluoro-1-propene (1234ze-E); et
   ii) un courant comprenant le 2,3,3,3-tétrafluoro-1-propène,

   c) récupération et séparation de ladite troisième composition pour former un courant comprenant ledit agent d'extraction organique et un courant comprenant ledit au moins un des composés sélectionné parmi le groupe consistant en 1,1-difluoroéthane (152a), chloropentafluoroéthane (115), trans-1,3,3,3-tetrafluoro-1-propene (1234ze-E) ; de préférence ledit agent d'extraction organique est recyclé à l'étape a).

2. Procédé selon la revendication précédente **caractérisé en ce que** la première composition comprend 2,3,3,3-tétrafluoro-1-propène, trans-1,3,3,3-tetrafluoro-1-propene (1234ze-E) et optionnellement ou non au moins un des composés sélectionné parmi le groupe consistant en 1,1-difluoroéthane (152a) et chloropentafluoroéthane (115) et la troisième composition comprend ledit agent d'extraction organique, trans-1,3,3,3-tetrafluoro-1-propene (1234ze-E) et optionnellement ou non au moins un des composés sélectionné parmi le groupe consistant en 1,1-difluoroéthane (152a) et chloropentafluoroéthane (115) ; l'étape c) étant la récupération de ladite troisième composition et séparation entre d'une part ledit agent d'extraction organique et d'autre part trans-1,3,3,3-tetrafluoro-1-propene (1234ze-E) et optionnellement ou non au moins un des composés sélectionné parmi le groupe consistant en 1,1-difluoroéthane (152a) et chloropentafluoroéthane (115).

3. Procédé selon l'une quelconque des revendications précédentes **caractérisé en ce que** ledit agent d'extraction organique est un solvant choisi parmi le groupe consistant en hydrocarbure, hydrohalocarbure, alcool, cétone, amine, ester, éther, aldéhyde, nitrile, carbonate, thioalkyle, amide et hétérocycle ; ou ledit agent d'extraction organique est difluorodiethylsilane, triethylfluorosilane ou l'acide perfluorobutanoïque; de préférence parmi le groupe consistant en amine, éther, cétone, ester, alcool, aldéhyde, hétérocycle.

4. Procédé selon l'une quelconque des revendications précédentes **caractérisé en ce que** le point d'ébullition dudit agent d'extraction organique est compris entre 10 et 150°C.

5. Procédé selon l'une quelconque des revendications précédentes **caractérisé en ce que** ledit agent d'extraction organique a un facteur de séparation $S_{1,2}$ supérieur ou égal à 1,1, ledit facteur de séparation étant calculé par la formule $S_{1,2} = (\gamma_{1,S} * P1)/(\gamma_{2,S} * P2)$ dans laquelle

   $\gamma_{1,S}$ représente le coefficient d'activité du 2,3,3,3-tétrafluoro-1-propène dans ledit agent d'extraction organique à dilution infinie,
   P1 représente la pression de vapeur saturante du 2,3,3,3-tétrafluoro-1-propène,
   $y_{2,s}$ représente le coefficient d'activité dudit au moins un des composés consistant en 1,1-difluoroéthane (152a), chloropentafluoroéthane (115), trans-1,3,3,3-tetrafluoro-1-propene (1234ze-E), dans ledit agent d'extraction organique à dilution infinie, de préférence $\gamma_{2,S}$ représente le coefficient d'activité du trans-1,3,3,3-tetrafluoro-1-propene (1234ze-E), dans ledit agent d'extraction organique à dilution infinie,
P2 représente la pression de vapeur saturante de dudit au moins un des composés consistant en 1,1-difluoroéthane (152a), chloropentafluoroéthane (115), trans-1,3,3,3-tetrafluoro-1-propene (1234ze-E), de préférence P2 représente la pression de vapeur saturante du trans-1,3,3,3-tetrafluoro-1-propene (1234ze-E) ;
avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

6. Procédé selon l'une quelconque des revendications précédentes **caractérisé en ce que** ledit agent d'extraction organique a une capacité d'absorption $C_{2,S}$ supérieure ou égale à 0,20, ladite capacité d'absorption étant calculé par la formule $C_{2,S} = 1/(\gamma_{2,S})$ dans laquelle $y_{2,s}$ représente le coefficient d'activité dudit au moins un des composés consistant en 1,1-difluoroéthane (152a), chloropentafluoroéthane (115), trans-1,3,3,3-tetrafluoro-1-propene (1234ze-E) dans ledit agent d'extraction organique à dilution infinie, de préférence $y_{2,s}$ représente le coefficient d'activité dudit au moins un des composés consistant en trans-1,3,3,3-tetrafluoro-1-propene (1234ze-E), dans ledit agent d'extraction organique à dilution infinie ;
avantageusement, la capacité d'absorption $C_{2,S}$ est supérieure ou égale à 0,40, de préférence supérieure ou égale à 0,60, plus préférentiellement supérieure ou égale à 0,80, en particulier supérieure ou égale à 1,0.

7. Procédé selon l'une quelconque des revendications précédentes **caractérisé en ce que** ladite première composition est une composition azéotropique ou quasi-azéotropique comprenant 2,3,3,3-tetrafluoro-1-propène, trans-1,3,3,3-tetrafluoro-1-propene (1234ze-E) et optionnellement ou non au moins un des composés sélectionné parmi le groupe consistant en 1,1-difluoroéthane (152a), chloropentafluoroéthane (115).

8. Procédé selon l'une quelconque des revendications précédentes **caractérisé en ce que** ledit agent d'extraction organique est choisi parmi le groupe consistant en éthylamine, isopropylamine, diethylether, ethoxy-ethene, dimethoxymethane, n-propylamine, methyl-t-butylether, diethylamine, propanone, methylacetate, isobutanal, tetrahydrofurane, isopropylformate, diisopropylether, 2-ethoxy-2-methyl-propane, ethylacetate, butanone, diethoxymethane, isopropylacetate, 3-pentylamine, 2-methoxyethanamine, tert-butylacetate, dioxane, 3-pentanone, 1,1-diethoxyethane, 2-pentanone, trimethoxymethane, n-pentylamine, 1,3-dioxane, 3,3-dimethyl-2-butanone, sec-butylacetate, 4-methyl-2-pentanone, 1,2-diaminoethane, 1-methoxy2-propanol, diethylcarbonate, n-butylacetate, 1-ethoxy-2-propanol, hexanal ; avantageusement ledit agent d'extraction organique est choisi parmi le groupe consistant en éthylamine, isopropylamine, diethylether, dimethoxymethane, n-propylamine, , diethylamine, , diisopropylether, 2-ethoxy-2-methyl-propane, butanone, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal ; de préférence ledit agent d'extraction organique est choisi parmi le groupe consistant en éthylamine, isopropylamine, diethylether, dimethoxymethane, n-propylamine, diethylamine, diisopropylether, 2-ethoxy-2-methyl-propane, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal.

9. Procédé selon l'une quelconque des revendications précédentes **caractérisé en ce que** le courant comprenant le 2,3,3,3-tetrafluoro-1-propène formé à l'étape b) est récupéré et est dépourvu d'au moins un des composés sélectionné parmi le groupe consistant en 1,1-difluoroéthane (152a), chloropentafluoroéthane (115), trans-1,3,3,3-tetrafluoro-1-propene (1234ze-E), de préférence est dépourvu de trans-1,3,3,3-tetrafluoro-1-propene (1234ze-E).

10. Procédé selon l'une quelconque des revendications précédentes **caractérisé en ce qu'**il comprend préalablement à l'étape a) les étapes :

i') mise en oeuvre d'une composition comprenant 2,3,3,3-tetrafluoro-1-propène, des impuretés ayant un point d'ébullition inférieur au point d'ébullition du 2,3,3,3-tetrafluoro-1-propène, et au moins un des composés sélectionné parmi le groupe consistant en 1,1-difluoroéthane (152a), chloropentafluoroéthane (115), trans-1,3,3,3-tetrafluoro-1-propene (1234ze-E), et optionnellement des impuretés lourdes ;
ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E), et optionnellement des impuretés lourdes, récupéré en bas de colonne de distillation ;

iii') optionnellement, distillation dudit premier courant récupéré en bas de colonne de distillation à l'étape ii') pour récupérer en tête de colonne un second courant comprenant du 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E), et en bas de colonne de distillation un courant comprenant les impuretés lourdes ;

ledit premier courant récupéré à l'étape ii') ou ledit second courant récupéré à l'étape iii') correspond à ladite première composition mise en oeuvre à l'étape a).

**11.** Procédé de production et de purification du 2,3,3,3-tetrafluoro-1-propène comprenant les étapes de :

A) fluoration en présence d'un catalyseur d'un composé de formule (I) $CX(Y)_2-CX(Y)_m-CH_mXY$ dans laquelle X et Y représentent indépendamment un atome d'hydrogène, de fluor ou de chlore et m = 0 ou 1; et/ou fluoration en présence d'un catalyseur d'un composé de formule $(CX_nY_{3-n})CH_pX_{1-p}CH_mX_{2-m}$ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;

B) récupération d'un courant comprenant du 2,3,3,3-tetrafluoro-1-propène, et au moins un des composés choisis parmi le groupe consistant en 1,1-difluoroéthane (152a), chloropentafluoroéthane (115), trans-1,3,3,3-tetrafluoro-1-propene (1234ze-E) ;

C) mise en oeuvre du procédé selon l'une quelconque des revendications 1 à 10 à partir du courant récupéré à l'étape B).

## Patentansprüche

1. Verfahren zur Reinigung von 2,3,3,3-Tetrafluor-1-propen (1234yf) ausgehend von einer ersten Zusammensetzung, die 2,3,3,3-Tetrafluor-1-propen und mindestens eine der Verbindungen aus der Gruppe bestehend aus 1,1-Difluorethan (152a), Chlorpentafluorethan (115) und trans-1,3,3,3-Tetrafluor-1-propen (1234ze-E) umfasst, wobei das Verfahren folgende Schritte umfasst:

a) Inkontaktbringen der ersten Zusammensetzung mit mindestens einem organischen Extraktionsmittel zur Bildung einer zweiten Zusammensetzung;

b) Extraktivdestillation der zweiten Zusammensetzung zur Bildung:

i) einer dritten Zusammensetzung, die das organische Extraktionsmittel und mindestens eine der Verbindungen aus der Gruppe bestehend aus 1,1-Difluorethan (152a), Chlorpentafluorethan (115) und trans-1,3,3,3-Tetrafluor-l-propen (1234ze-E) umfasst; und

ii) eines Stroms, der 2,3,3,3-Tetrafluor-1-propen umfasst;

c) Gewinnung und Trennung der dritten Zusammensetzung zur Bildung eines Stroms, der das organische Extraktionsmittel umfasst, und eines Stroms, der mindestens eine der Verbindungen aus der Gruppe bestehend aus 1,1-Difluorethan (152a), Chlorpentafluorethan (115) und trans1,3,3,3-Tetrafluor-1-propen (1234ze-E) umfasst; wobei das organische Extraktionsmittel vorzugsweise zu Schritt a) zurückgeführt wird.

2. Verfahren nach dem vorhergehenden Anspruch, **dadurch gekennzeichnet, dass** die erste Zusammensetzung 2,3,3,3-Tetrafluor-1-propen, trans-1,3,3,3-Tetrafluor-1-propen (1234ze-E) und gegebenenfalls oder nicht mindestens eine der Verbindungen aus der Gruppe bestehend aus 1,1-Difluorethan (152a) und Chlorpentafluorethan (115) umfasst und die dritte Zusammensetzung das organische Extraktionsmittel, trans-1,3,3,3-Tetrafluor-1-propen (1234ze-E) und gegebenenfalls oder nicht mindestens eine der Verbindungen aus der Gruppe bestehend aus 1,1-Difluorethan (152a) und Chlorpentafluorethan (115) umfasst; wobei es sich bei Schritt c) um die Gewinnung der dritten Zusammensetzung und Trennung zwischen dem organischen Extraktionsmittel einerseits und trans-1,3,3,3-Tetrafluor-1-propen (1234ze-E) und gegebenenfalls oder nicht mindestens einer der Verbindungen aus der Gruppe bestehend aus 1,1-Difluorethan (152a) und Chlorpentafluorethan (115) andererseits handelt.

3. Verfahren nach einem der vorhergehenden Ansprüche, **dadurch gekennzeichnet, dass** das organische Extraktionsmittel ein Lösungsmittel aus der Gruppe bestehend aus Kohlenwasserstoff, Halogenkohlenwasserstoff, Alkohol, Keton, Amin, Ester, Ether, Aldehyd, Nitril, Carbonat, Thioalkyl, Amid und Heterocyclus ist oder das organische Extraktionsmittel Difluordiethylsilan, Triethylfluorsilan oder Perfluorbutansäure ist und vorzugsweise aus der Gruppe bestehend aus Amin, Ether, Keton, Ester, Alkohol, Aldehyd und Heterocyclus ausgewählt wird.

4. Verfahren nach einem der vorhergehenden Ansprüche, **dadurch gekennzeichnet, dass** der Siedepunkt des organischen Extraktionsmittels zwischen 10 und 150°C liegt.

5. Verfahren nach einem der vorhergehenden Ansprüche, **dadurch gekennzeichnet, dass** das organische Extraktionsmittel einen Trennfaktor $S_{1,2}$ größer oder gleich 1,1 aufweist, wobei der Trennfaktor anhand der Formel $S_{1,2} = (\gamma_{1,S}*P1) / (\gamma_{2,S}*P2)$ berechnet wird, wobei

$\gamma_{1,S}$ für den Aktivitätskoeffizienten von 2,3,3,3-Tetrafluor-1-propen in dem organischen Extraktionsmittel bei unendlicher Verdünnung steht,
P1 für den Sättigungsdampfdruck von 2,3,3,3-Tetrafluor-1-propen steht,
$\gamma_{2,S}$ für den Aktivitätskoeffizienten der mindestens einen der aus 1,1-Difluorethan (152a), Chlorpentafluorethan (115) und trans-1,3,3,3-Tetrafluor-1-propen (1234ze-E) bestehenden Verbindungen in dem organischen Extraktionsmittel bei unendlicher Verdünnung steht und $\gamma_{2,S}$ vorzugsweise für den Aktivitätskoeffizienten von trans-1,3,3,3-Tetrafluor-1-propen (1234ze-E) in dem organischen Extraktionsmittel bei unendlicher Verdünnung steht,
P2 für den Sättigungsdampfdruck der mindestens einen der aus 1,1-Difluorethan (152a), Chlorpentafluorethan (115) und trans-1,3,3,3-Tetrafluor-1-propen (1234ze-E) bestehenden Verbindungen steht und P2 vorzugsweise für den Sättigungsdampfdruck von trans-1,3,3,3-Tetrafluor-1-propen (1234ze-E) steht; der Trennfaktor vorteilhafterweise größer oder gleich 1,2, vorzugsweise größer oder gleich 1,4, weiter bevorzugt größer oder gleich 1,6, insbesondere größer oder gleich 1,8 und spezieller größer oder gleich 2,0 ist.

6. Verfahren nach einem der vorhergehenden Ansprüche, **dadurch gekennzeichnet, dass** das organische Extraktionsmittel eine Absorptionskapazität $C_{2,S}$ größer oder gleich 0,20 aufweist, wobei die Absorptionskapazität anhand der Formel $C_{2,S} = 1/ (\gamma_{2,S})$ berechnet wird, wobei $\gamma_{2,S}$ für den Aktivitätskoeffizienten der mindestens einen der aus 1,1-Difluorethan (152a), Chlorpentafluorethan (115) und trans-1,3,3,3-Tetrafluor-1-propen (1234ze-E) bestehenden Verbindungen in dem organischen Extraktionsmittel bei unendlicher Verdünnung steht und $\gamma_{2,S}$ vorzugsweise für den Aktivitätskoeffizienten der mindestens einen der aus trans-1,3,3,3-Tetrafluor-1-propen (1234ze-E) bestehenden Verbindungen in dem organischen Extraktionsmittel bei unendlicher Verdünnung steht; die Absorptionskapazität $C_{2,S}$ vorteilhafterweise größer oder gleich 0,40, vorzugsweise größer oder gleich 0,60, weiter bevorzugt größer oder gleich 0,80 und insbesondere größer oder gleich 1,0 ist.

7. Verfahren nach einem der vorhergehenden Ansprüche, **dadurch gekennzeichnet, dass** es sich bei der ersten Zusammensetzung um eine azeotrope oder quasiazeotrope Zusammensetzung handelt, die 2,3,3,3-Tetrafluor-1-propen, trans-1,3,3,3-Tetrafluor-1-propen (1234ze-E) und gegebenenfalls oder nicht mindestens eine der Verbindungen aus der Gruppe bestehend aus 1,1-Difluorethan (152a) und Chlorpentafluorethan (115) umfasst.

8. Verfahren nach einem der vorhergehenden Ansprüche, **dadurch gekennzeichnet, dass** das organische Extraktionsmittel aus der Gruppe bestehend aus Ethylamin, Isopropylamin, Diethylether, Ethoxyethen, Dimethoxymethan, n-Propylamin, Methylt-butylether, Diethylamin, Propanon, Methylacetat, Isobutanal, Tetrahydrofuran, Isopropylformiat, Diisopropylether, 2-Ethoxy-2-methylpropan, Ethylacetat, Butanon, Diethoxymethan, Isopropylacetat, 3-Pentylamin, 2-Methoxyethanamin, tert-Butylacetat, Dioxan, 3-Pentanon, 1,1-Diethoxyethan, 2-Pentanon, Trimethoxymethan, n-Pentylamin, 1,3-Dioxan, 3,3-Dimethyl-2-butanon, sec-Butylacetat, 4-Methyl-2-pentanon, 1,2-Diaminoethan, 1-Methoxy-2-propanol, Diethylcarbonat, n-Butylacetat, 1-Ethoxy-2-propanol und Hexanal ausgewählt wird; das organische Extraktionsmittel vorteilhafterweise aus der Gruppe bestehend aus Ethylamin, Isopropylamin, Diethylether, Dimethoxymethan, n-Propylamin, Diethylamin, Diisopropylether, 2-Ethoxy-2-methylpropan, Butanon, Diethoxymethan, Isopropylacetat, 3-Pentylamin, 2-Methoxyethanamin, tert-Butylacetat, Dioxan, Trimethoxymethan, n-Pentylamin, 1,3-Dioxan, sec-Butylacetat, 1,2-Diaminoethan, 1-Methoxy-2-propanol, n-Butylacetat, 1-Ethoxy-2-propanol und Hexanal ausgewählt wird; das organische Extraktionsmittel vorzugsweise aus der Gruppe bestehend aus Ethylamin, Isopropylamin, Diethylether, Dimethoxymethan, n-Propylamin, Diethylamin, Diisopropylether, 2-Ethoxy-2-methylpropan, Diethoxymethan, Isopropylacetat, 3-Pentylamin, 2-Methoxyethanamin, tert-Butylacetat, Dioxan, Trimethoxymethan, n-Pentylamin, 1,3-Dioxan, sec-Butylacetat, 1,2-Diaminoethan, 1-Methoxy-2-propanol, n-Butylacetat, 1-Ethoxy-2-propanol und Hexanal ausgewählt wird.

9. Verfahren nach einem der vorhergehenden Ansprüche, **dadurch gekennzeichnet, dass** der in Schritt b) gebildete Strom, der 2,3,3,3-Tetrafluor-1-propen umfasst, gewonnen wird und frei von mindestens einer der Verbindungen aus der Gruppe bestehend aus 1,1-Difluorethan (152a), Chlorpentafluorethan (115) und trans-1,3,3,3-Tetrafluor-1-propen (1234ze-E), vorzugsweise frei von trans-1,3,3,3-Tetrafluor-1-propen (1234ze-E), ist.

**10.** Verfahren nach einem der vorhergehenden Ansprüche, **dadurch gekennzeichnet, dass** es vor Schritt a) folgende Schritte umfasst:

i') Verwendung einer Zusammensetzung, die 2,3,3,3-Tetrafluor-1-propen, Verunreinigungen mit einem unter dem Siedepunkt von 2,3,3,3-Tetrafluor-1-propen liegenden Siedepunkt und mindestens eine der Verbindungen aus der Gruppe bestehend aus 1,1-Difluorethan (152a), Chlorpentafluorethan (115) und trans-1,3,3,3-Tetrafluor-1-propen (1234ze-E) und gegebenenfalls schwere Verunreinigungen umfasst;

ii') Destillation der Zusammensetzung aus Schritt i) zur Entfernung von Verunreinigungen mit einem unter dem Siedepunkt von 2,3,3,3-Tetrafluor-1-propen liegenden Siedepunkt am Kopf der Säule und zur Bildung eines ersten Stroms, der 2,3,3,3-Tetrafluor-1-propen und mindestens eine der Verbindungen aus der Gruppe bestehend aus 1,1-Difluorethan (152a), Chlorpentafluorethan (115) und trans-1,3,3,3-Tetrafluor-1-propen (1234ze-E) und gegebenenfalls schwere Verunreinigungen umfasst und am Sumpf der Destillationssäule gewonnen wird;

iii') gegebenenfalls Destillation des am Sumpf der Destillationssäule in Schritt ii') gewonnenen ersten Stroms zur Gewinnung eines zweiten Stroms, der 2,3,3,3-Tetrafluor-1-propen und mindestens eine der Verbindungen aus der Gruppe bestehend aus 1,1-Difluorethan (152a), Chlorpentafluorethan (115) und trans-1,3,3,3-Tetrafluor-1-propen (1234ze-E) umfasst, am Kopf der Säule und eines Stroms, der die schweren Verunreinigungen umfasst, am Sumpf der Destillationssäule;

wobei der in Schritt ii') gewonnene erste Strom oder der in Schritt iii') gewonnene zweite Strom der in Schritt a) verwendeten ersten Zusammensetzung entspricht.

**11.** Verfahren zur Herstellung und Reinigung von 2,3,3,3-Tetrafluor-1-propen, das folgende Schritte umfasst:

A) Fluorierung einer Verbindung der Formel (I) $CX(Y)_2$-$CX(Y)_m$-$CH_mXY$, in der X und Y unabhängig für ein Wasserstoff-, Fluor- oder Chloratom stehen und m = 0 oder 1, in Gegenwart eines Katalysators und/oder Fluorierung einer Verbindung der Formel $(CX_nY_{3-n})CH_pX_{1-p}CH_mX_{2-m}$ (II), in der X unabhängig voneinander für Cl, F, I oder Br steht; Y unabhängig voneinander für H, Cl, F, I oder Br steht; n für 1, 2 oder 3 steht und m für 0, 1 oder 2 steht und p für 0 oder 1 steht, in Gegenwart eines Katalysators;

B) Gewinnung eines Stroms, der 2,3,3,3-Tetrafluor-1-propen und mindestens eine der Verbindungen aus der Gruppe bestehend aus 1,1-Difluorethan (152a), Chlorpentafluorethan (115) und trans-1,3,3,3-Tetrafluor-1-propen (1234ze-E) umfasst;

C) Durchführung des Verfahrens nach einem der Ansprüche 1 bis 10 ausgehend von dem in Schritt B) gewonnenen Strom.

**Claims**

**1.** Process for purifying 2,3,3,3-tetrafluoro-1-propene (1234yf) using a first composition comprising 2,3,3,3-tetrafluoro-1-propene and at least one of the compounds chosen from the group consisting of 1,1-difluoroethane (152a), chloropentafluoroethane (115) and trans-1,3,3,3-tetrafluoro-1-propene (1234ze-E), said process comprising the steps of:

a) placing said first composition in contact with at least one organic extracting agent to form a second composition;
b) extractive distillation of said second composition to form:

i) a third composition comprising said organic extracting agent and said at least one compound chosen from the group consisting of 1,1-difluoroethane (152a), chloropentafluoroethane (115) and trans-1,3,3,3-tetrafluoro-1-propene (1234ze-E); and
ii) a stream comprising 2,3,3,3-tetrafluoro-1-propene;

c) recovery and separation of said third composition to form a stream comprising said organic extracting agent and a stream comprising said at least one compound chosen from the group consisting of 1,1-difluoroethane (152a), chloropentafluoroethane (115) and trans-1,3,3,3-tetrafluoro-l-propene (1234ze-E); preferably, said organic extracting agent is recycled into step a).

**2.** Process according to the preceding claim, **characterized in that** the first composition comprises 2,3,3,3-tetrafluoro-1-propene, trans-1,3,3,3-tetrafluoro-1-propene (1234ze-E) and optionally or not at least one of the compounds chosen from the group consisting of 1,1-difluoroethane (152a) and chloropentafluoroethane (115) and the third composition comprises said organic extracting agent, trans-1,3,3,3-tetrafluoro-l-propene (1234ze-E) and optionally or not at least one of the compounds chosen from the group consisting of 1,1-difluoroethane (152a) and chloropentafluoroethane (115); step c) being the recovery of said third composition and separation between, on the one hand, said organic extracting agent and, on the other hand, trans-1,3,3,3-tetrafluoro-1-propene (1234ze-E) and optionally or not at least one of the compounds chosen from the group consisting of 1,1-difluoroethane (152a) and chloropentafluoroethane (115) .

3. Process according to either of the preceding claims, **characterized in that** said organic extracting agent is a solvent chosen from the group consisting of hydrocarbon, halohydrocarbon, alcohol, ketone, amine, ester, ether, aldehyde, nitrile, carbonate, thioalkyl, amide and heterocycle; or said organic extracting agent is difluorodiethylsilane, triethylfluorosilane or perfluorobutanoic acid; preferably from the group consisting of amine, ether, ketone, ester, alcohol, aldehyde and heterocycle.

4. Process according to any one of the preceding claims, **characterized in** the boiling point of said organic extracting agent is between 10 and 150°C.

5. Process according to any one of the preceding claims, **characterized in that** said organic extracting agent has a separation factor $S_{1,2}$ of greater than or equal to 1.1, said separation factor being calculated by the formula $S_{1,2} = (\gamma_{1,S}*P1) / (\gamma_{2,S}*P2)$ in which

$\gamma_{1,S}$ represents the activity coefficient of 2,3,3,3-tetrafluoro-1-propene in said organic extracting agent at infinite dilution;
P1 represents the saturating vapor pressure of 2,3,3,3-tetrafluoro-1-propene;
$\gamma_{2,S}$ represents the activity coefficient of said at least one compound consisting of 1,1-difluoroethane (152a), chloropentafluoroethane (115) and trans-1,3,3,3-tetrafluoro-1-propene (1234ze-E) in said organic extracting agent at infinite dilution, preferably, $\gamma_{2,S}$ represents the activity coefficient of trans-1,3,3,3-tetrafluoro-1-propene (1234ze-E) in said organic extracting agent at infinite dilution;
P2 represents the saturating vapor pressure of said at least one compound consisting of 1,1-difluoroethane (152a), chloropentafluoroethane (115) and trans-1,3,3,3-tetrafluoro-l-propene (1234ze-E), preferably, P2 represents the saturating vapor pressure of trans-1,3,3,3-tetrafluoro-1-propene (1234ze-E);
advantageously, the separation factor is greater than or equal to 1.2, preferably greater than or equal to 1.4, more preferentially greater than or equal to 1.6, in particular greater than or equal to 1.8, more particularly greater than or equal to 2.0.

6. Process according to any one of the preceding claims, **characterized in that** said organic extracting agent has an absorption capacity $C_{2,S}$ of greater than or equal to 0.20, said absorption capacity being calculated by the formula $C_{2,S} = 1/(\gamma_{2,S})$ in which $\gamma_{2,S}$ represents the activity coefficient of said at least one compound consisting of 1,1-difluoroethane (152a), chloropentafluoroethane (115) and trans-1,3,3,3-tetrafluoro-1-propene (1234ze-E) in said organic extracting agent at infinite dilution; preferably, $\gamma_{2,s}$ represents the activity coefficient of said at least one compound consisting of trans-1,3,3,3-tetrafluoro-1-propene (1234ze-E), in said organic extracting agent at infinite dilution;
advantageously, the absorption capacity $C_{2,S}$ is greater than or equal to 0.40, preferably greater than or equal to 0.60, more preferentially greater than or equal to 0.80, in particular greater than or equal to 1.0.

7. Process according to any one of the preceding claims, **characterized in that** said first composition is an azeotropic or quasi-azeotropic composition comprising 2,3,3,3-tetrafluoro-1-propene, trans-1,3,3,3-tetrafluoro-1-propene (1234ze-E) and optionally or not at least one compound chosen from the group consisting of 1,1-difluoroethane (152a) and chloropentafluoroethane (115).

8. Process according to any one of the preceding claims, **characterized in that** said organic extracting agent is chosen from the group consisting of ethylamine, isopropylamine, diethyl ether, ethoxyethene, dimethoxymethane, n-propylamine, methyl t-butyl ether, diethylamine, propanone, methyl acetate, isobutanal, tetrahydrofuran, isopropyl formate, diisopropyl ether, 2-ethoxy-2-methylpropane, ethyl acetate, butanone, diethoxymethane, isopropyl acetate, 3-pentylamine, 2-methoxyethanamine, tert-butyl acetate, dioxane, 3-pentanone, 1,1-diethoxyethane, 2-pentanone, trimethoxymethane, n-pentylamine, 1,3-dioxane, 3,3-dimethyl-2-butanone, sec-butyl acetate, 4-methyl-2-pentanone, 1,2-diaminoethane, 1-methoxy2-propanol, diethyl carbonate, n-butyl acetate, 1-ethoxy-2-propanol and hexanal; advantageously, said organic extracting agent is chosen from the group consisting of ethylamine, isopropylamine, diethyl ether, dimethoxymethane, n-propylamine, diethylamine, diisopropyl ether, 2-ethoxy-2-methylpropane, butanone, diethoxymethane, isopropyl acetate, 3-pentylamine, 2-methoxyethanamine, tert-butyl acetate, dioxane, trimethoxymethane, n-pentylamine, 1,3-dioxane, sec-butyl acetate, 1,2-diaminoethane, 1-methoxy-2-propanol, n-butyl acetate, 1-ethoxy-2-propanol and hexanal; preferably, said organic extracting agent is chosen from the group consisting of ethylamine, isopropylamine, diethyl ether, dimethoxymethane, n-propylamine, diethylamine, diisopropyl ether, 2-ethoxy-2-methylpropane, diethoxymethane, isopropyl acetate, 3-pentylamine, 2-methoxyethanamine, tert-butyl acetate, dioxane, trimethoxymethane, n-pentylamine, 1,3-dioxane, sec-butyl acetate, 1,2-diaminoethane, 1-methoxy-2-propanol, n-butyl acetate, 1-ethoxy-2-propanol and hexanal.

9. Process according to any one of the preceding claims, **characterized in that** the stream comprising 2,3,3,3-tetrafluoro-1-propene formed in step b) is recovered and is freed of at least one of the compounds chosen from the group consisting of 1,1-difluoroethane (152a), chloropentafluoroethane (115) and trans-1,3,3,3-tetrafluoro-1-propene (1234ze-E), preferably freed of trans-1,3,3,3-tetrafluoro-1-propene (1234ze-E).

10. Process according to any one of the preceding claims, **characterized in that** it comprises, prior to step a), the following steps:

i') use of a composition comprising 2,3,3,3-tetrafluoro-1-propene, impurities with a boiling point below the boiling point of 2,3,3,3-tetrafluoro-1-propene, and at least one compound chosen from the group consisting of 1,1-difluoroethane (152a), chloropentafluoroethane (115) and trans-1,3,3,3-tetrafluoro-1-propene (1234ze-E), and optionally heavy impurities;

ii') distillation of said composition from step i) to remove, at the top of the column, impurities with a boiling point below the boiling point of 2,3,3,3-tetrafluoro-1-propene and to form a first stream comprising 2,3,3,3-tetrafluoro-1-propene and at least one compound chosen from the group consisting of 1,1-difluoroethane (152a), chloropentafluoroethane (115) and trans-1,3,3,3-tetrafluoro-1-propene (1234ze-E), and optionally heavy impurities, recovered at the bottom of the distillation column;

iii') optionally, distillation of said first stream recovered at the bottom of the distillation column in step ii') to recover, at the top of the column, a second stream comprising 2,3,3,3-tetrafluoro-1-propene and at least one compound chosen from the group consisting of 1,1-difluoroethane (152a), chloropentafluoroethane (115) and trans-1,3,3,3-tetrafluoro-1-propene (1234ze-E), and, at the bottom of the distillation column, a stream comprising the heavy impurities;

said at first stream recovered in step ii') or said second stream recovered in step iii') corresponds to said first composition used in step a).

11. Process for producing and purifying 2,3,3,3-tetrafluoro-1-propene, comprising the steps of:

A) fluorination in the presence of a catalyst for a compound of formula (I) $CX(Y)_2$-$CX(Y)_m$-$CH_mXY$ in which X and Y independently represent a hydrogen, fluorine or chlorine atom and m = 0 or 1; and/or fluorination in the presence of a catalyst for a compound of formula $(CX_nY_{3-n})CH_pX_{1-p}CH_mX_{2-m}$ (II) in which X is, independently of each other, Cl, F, I or Br; Y is, independently of each other, H, Cl, F, I or Br; n is 1, 2 or 3; and m is 0, 1 or 2; and p is 0 or 1;

B) recovery of a stream comprising 2,3,3,3-tetrafluoro-1-propene and at least one compound chosen from the group consisting of 1,1-difluoroethane (152a), chloropentafluoroethane (115) and trans-1,3,3,3-tetrafluoro-l-propene (1234ze-E);

C) implementation of the process according to any one of Claims 1 to 10 using the stream recovered in step B).

FIG. 1a

EP 3 394 014 B1

FIG. 1b

FIG. 1c

FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0864554 A
- WO 03068716 A
- WO 9819982 A
- WO 2009105521 A
- WO 2007079431 A
- EP 939071 A
- WO 2008054781 A
- WO 2008040969 A
- US 4902838 A
- WO 2009118628 A